# EUROPEAN PATENT APPLICATION

(11) **EP 2 161 031 A1**
(43) Date of publication of application: **10.03.2010**
(21) Application number: 08163800.9
(22) Date of filing: 05.09.2008
(51) Int. Cl.: A61K 38/17

(54) **Fc gamma receptor for the treatment of B cell mediated multiple sclerosis**

(71) Applicant: SuppreMol GmbH, 82152 Martinsried/München (DE)
(72) Inventor: The designation of the inventor has not yet been filed
(74) Representative: Vossius & Partner

(57) **Abstract**

The invention relates to the Fcγ receptor (Fc-gamma receptor) for use in treating multiple sclerosis, wherein the multiple sclerosis is a B cell mediated form of multiple sclerosis and/or an autoantibody driven form of multiple sclerosis. The invention relates to pharmaceutical compositions containing the Fcγ receptor (Fc-gamma receptor) for use in treating multiple sclerosis, wherein the multiple sclerosis is a B cell mediated form of multiple sclerosis and/or an autoantibody driven form of multiple sclerosis.

## Description

### FIELD OF THE INVENTION

The invention is in the field of biotechnology and therapeutics. The invention relates to the Fcγ receptor (Fe-gamma receptor) for use in treating multiple sclerosis, wherein the multiple sclerosis is a B cell mediated form of multiple sclerosis and/or an autoantibody driven form of multiple sclerosis.

### BACKGROUND OF THE INVENTION

Current hypotheses favour the concept that T cells play a pivotal role in the pathogenesis of Multiple Sclerosis (MS), which was initially based upon the observation that T cells are the predominant lymphocyte class present in MS lesions (Windhagen, et al., Cytokine, secretion of myelin basic protein reactive T cells in patients with multiple sclerosis. Journal of Neuroimmunology, 91:1-9, 1998; Hafler, D.A., et al., Oral administration of myelin induces antigen-specific TGF-beta 1 secreting cells in patients with multiple sclerosis. Annals of the New York Academy of Science, 835:120-131, 1997; Lovett-Racke, A.E., et al., Decreased dependence of myelin basic protein-reactive T cells on CD28-mediated costimulaftion in multiple sclerosis patients, Journal of Clinical Investigation, 101:725-730, 1998) This observation continues to be a cardinal hallmark of the disease, and is supported by a number of observations. For example, active CD4+ T helper cells bearing anti-myelin T Cell Receptors (TCRs) are present in the CSF of patients with MS. In addition, elevated levels of Th1-like cytokines have been detected in the CSF of patients with MS and have been correlated with worsening of the disease in some cases (Calabresi et al, Cytokine expression in cells derived from CSF of multiple sclerosis patients. Journal of Neuroimmunology, 89:198-205, 1998).

There has, however, also been evidence that B cells may be involved in the development and perpetuation of MS including:
(1) elevated immunoglobulin levels in the CSF of MS patients (Link, H., et al., Immunoglobulins in multiple sclerosis and infections of the nervous system, Archives of Neurology, 25:326-344, 1971; Link, H., et al., Immunoglobulin class and light chain type of oligoclonal bands in CSF in multiple sclerosis determined by agarose gel electrophoresis and immunofixation. Ann Neurol, 6(2):107-110, 1979; Perez, L., et al., B cells capable of spontaneous IgG secretion in cerebrospinal fluid from patients with multiple sclerosis: dependancy on local IL-6 production. Clinical Experimental Immunology, 101:449-452, 1995),
(2) oligoclonal banding in the CSF of MS patients (Link, H., et al., Immunoglobulin class and light chain type of oligoclonal bands in CSF in multiple sclerosis determined by agarose gel electrophoresis and immunofixation. Ann Neurol, 6(2): 107-110, 1979),
(3) skewing of the κ:λ ratio in the CSF of MS patients (Hauser, S.L., et al., Clonally restricted B cells in peripheral blood of multiple sclerosis patients: kappa/lambda staining patterns. Annals of Neurology, 11:408-412,1982),
(4) the presence of anti-myelin antibodies in the CSF of MS patients (Sun, J. H., et al, B cell responses to myelin-oligodendrocyte glycoprotein in multiple sclerosis. Journal of Immunology, 146:1490-1495, 1991) and
(5) the demonstration that antibodies from the CSF of MS patients may contribute to the overall extent of tissue injury in these patients (Lassmann, H., et al., Experimental allergic encephalojyelitis: the balance between encephalitogenic T lymphocytes and demyelinating antibodies determines size and structure of demyelinated lesions. Acta Neuropathology, 75:566-576,1988).

A number of publications demonstrate such B cell mediation: Bourquin, et al., The journal of Immunology, 2003, 171: 455-461; Stromnes et al Nature Protocols, Vol. 1, No. 4, 2006: 1810-1818; Stromnes et al Nature Protocols, Vol. 1, No. 4, 2006: 1952-19160; Oliver, et al., The Journal of Immunology, 2003, 171: 462-468;

The basic role of B cells in the immune system is to mediate Humoral Immune responses. That is, to secrete proteins called antibodies (or immunoglobulin) that bind to foreign bodies and mark them for elimination from the body by other immune cells such as NK cells and macrophages.

Intravenous immune globulin IVIG has been demonstrated in multiple clinical trials reported in the medical literature to have an impact on two important considerations in relapsing-remitting Multiple Sclerosis. IVIG reduces the frequency of the acute exacerbation and it reduces the intensity and duration of the acute exacerbation. Seemingly, a subset of patients has a prominent B cell involvement in multiple sclerosis. Hence, it would be advantageous to have substances and methods for the treatment of such B cell mediated multiple sclerosis patients.

### SUMMARY OF THE INVENTION

The inventors have astonishingly found that the current hypothesis that T cells play a pivotal role in the pathogenesis of Multiple Sclerosis (MS) is at least not entirely correct. The inventors addressed the B cell mediated form of multiple sclerosis and are, astonishingly able to provide for a special treatment.

The invention relates to the Fcγ receptor (Fc-gamma receptor) for use in treating multiple sclerosis, wherein the multiple sclerosis is a B cell mediated form of multiple sclerosis and/or an autoantibody driven form of multiple sclerosis. The invention relates to pharmaceutical compositions containing the Fcγ receptor (Fc-gamma receptor) for use in treating multiple sclerosis, wherein the multiple sclerosis is a B cell mediated form of multiple sclerosis and/or an autoantibody driven form of multiple sclerosis.

Fc receptors (FcRs) play a key role in defending the human organism against infections. After pathogens have gained access to the blood circulation they are opsonized by immunoglobulins (Igs). The resulting immunocomplexes bind due to their multivalency with high avidity to FcR bearing cells leading to clustering of the FcRs, which triggers several effector functions (Metzger, H., 1992A). These include, depending on the expressed FcR type and associated proteins, endocytosis with subsequent neutralization of the pathogens and antigen presentation, antibodydependent cellular cytotoxity (ADCC), secretion of mediators or the regulation of antibody production (Fridman, *et al*., 1992; van de Winkel and Capel, 1993).

Specific FcRs exist for all lg classes, the ones for IgG being the most abundant with the widest diversity. Together with the high affinity receptor for IgE (FcεRIa), FcγRI (CD64), FcγRII (CD32) and FcγRIIIa (CD16) occur as type I transmembrane proteins or in soluble forms (sFcRs) but also a glycosylphosphatidylinositol anchored form of the FcγRIII (FcγRIIIb) exists. Furthermore, FcγRs occur in various isoforms (FcγRIa, b1, b2, c; FcγRIIa1-2, b1-3, c) and alleles (FcγRIIa1-HR, -LR; FcyRIIIb-NA1 ,-DNA2) (van de Winkel and Capel, 1993). In contrast to the overall homologous extracellular parts, the membrane spanning and the cytoplasmic domains differ. They may be deleted entirely or be of a size of 8 kDa. They may contain either a 26 amino acid immunoreceptor tyrosine-based activation motif (ITAM) as in FcγRIIa or a respective 13 amino acid inhibitory motif (ITIM) in FcγRIIb involved in signal transduction (Amigorena, *et al.,* 1992).

Herein, EAE is experimental autoimmune encephalomyelitis.

### DETAILED DESCRIPTION OF THE INVENTION

The invention relates to the Fcγ receptor (Fc-gamma receptor) for use in treating multiple sclerosis, wherein the multiple sclerosis is a B cell mediated form of multiple sclerosis and/or an autoantibody driven form of multiple sclerosis.

The B cell mediation of the multiple sclerosis and/or autoantibody driven form of multiple sclerosis is characterized by one or more of the following features, (a) the multiple sclerosis is ameliorated if the patient undergoes Intravenous immunoglobulin (IVIG) treatment and/or, (b) the multiple sclerosis is ameliorated if the patient undergoes anti-CD20 antibody treatment and/or, (c) the multiple sclerosis is ameliorated if the patient undergoes plasmapheresis and/or, (d) the presence of auto-antibodies against the antigen Myelin Oligodendrocyte Glycoprotein (MOG) and/or (e) the presence of auto-antibodies against the antigen myelin basic protein (MBP) and/or the presence of auto-antibodies against aquaporin 4.

Devic's disease is similar to MS in that the body's immune system attacks the myelin surrounding nerve cells. Unlike standard MS, the attacks are not believed to be mediated by the immune system's T cells but rather by antibodies called NMO-IgG. These antibodies target a protein called aquaporin 4 in the cell membranes of astrocytes which acts as a channel for the transport of water across the cell membrane.

In a preferred embodiment the B cell mediation of the multiple sclerosis and/or autoantibody driven form of multiple sclerosis is determined prior to the use of the Fcγ receptor by means of one or more of the following tests, (a) determining whether the multiple sclerosis is ameliorated if the patient undergoes Intravenous immunoglobulin (IVIG) treatment and/or, (b) the multiple sclerosis is ameliorated if the patient undergoes anti-CD20 antibody treatment and/or, (c) the multiple sclerosis is ameliorated if the patient undergoes plasmapheresis, (d) determining whether auto-antibodies against the antigen myelin oligodendrocyte glycoprotein (MOG) are present in the patient and/or, (e) determining the presence of auto-antibodies against aquaporin 4. Preferably a selection of two of the test are performed, more preferably a selection of three of tests are performed, more preferably a selection of four of the tests are performed.

One example of an anti-CD20 treatment is Rituximab. This is monoclonal antibody. Sadly, it has severe side-effects which could be ameliorated if the present protein, *i*.*e*. polypetides were used in place of Rituximab.

During plasmapheresis, blood is initially taken out of the body through a needle or previously implanted catheter. Plasma is then removed from the blood by a cell separator. Three procedures are commonly used to separate the plasma from the blood:
Discontinuous flow centrifugation:
   One venous catheter line is required. Typically, a 300 ml batch of blood is removed at a time and centrifuged to separate plasma from blood cells.
Continuous flow centrifugation
   Two venous lines are used. This method requires slightly less blood volume to be out of the body at any one time as it is able to continuously spin out plasma.
Plasma filtration
   Two venous lines are used. The plasma is filtered using standard hemodialysis equipment. This continuous process requires less than 100 ml of blood to be outside the body at one time.

Each method has its advantages and disadvantages. After plasma separation, the blood cells are returned to the person undergoing treatment, while the plasma, which contains the antibodies, is first treated and then returned to the patient in traditional plasmapheresis. (In plasma exchange, the removed plasma is discarded and the patient receives replacement donor plasma or saline with added proteins.) Medication to keep the blood from clotting (an anticoagulant) is generally given to the patient during the procedure. Plasmapheresis is used as a therapy in particular diseases.

An important use of plasmapheresis is in the therapy of autoimmune disorders. However, the method is extremely strenuous for the patient.

WO°2008/017363 discloses means for testing for B cell mediation. In particular it discloses means of detecting auto-antibodies against MOG and aquaporin 4. WO°2008/017363 is incorporated by reference.

In a preferred embodiment of the invention the FcR receptor is of human origin. The Fcγ receptor according to the invention is preferably selected from the group of, FcγRI (CD64), FcγRIIA (CD32), FcγRIIB1 (CD32), FcγRIIB2 (CD32), FcγRIIc (CD32), FcγRIIIA (CD16) and FcγRIIIB (CD16).

FcγRIIB1 (CD32) and FcγRIIB2 (CD32) are so called isoforms, i.e. the isoforms 1 and 2.

According to the present invention, the preparation of the soluble Fc receptors takes place in prokaryotic or eukaryotic cells. It may also take place in eukaryotic cells. If it takes place in prokaryotic cells (see EP-B1 1 135 486) insoluble inclusion bodies containing the recombinant protein form, thus facilitating purification by separation of the inclusion bodies from other cell components before renaturation of the proteins contained therein takes place. The renaturation of the FcRs according to the present invention which are contained in the inclusion bodies can principally take place according to known methods. The advantage of the preparation in prokaryotic cells, the production of inclusion bodies and the thus obtained recombinant soluble Fc receptors make it possible to obtain a very pure and, in particular, also very homogeneous FcR preparation. Also because of the absence of glycosylation the obtained product is of great homogeneity. However, in some cases glycosylation may be desired.
A host cell is genetically engineered with the polynucleotide or the vector encoding or carrying the FcR. The host cells that may be used for purposes of the invention include but are not limited to prokaryotic cells such as bacteria (for example, *E*. *coli* and *B. subtilis*), which can be transformed with, for example, recombinant bacteriophage DNA, plasmid DNA, or cosmid DNA expression vectors containing the polynucleotide molecules encoding the FcR; simple eukaryotic cells like yeast (for example, *Saccharomyces* and *Pichia*), which can be transformed with, for example, recombinant yeast expression vectors containing the polynucleotide molecule of the invention, *i*.*e*. the polynucleotide molecules encoding the FcR; insect cell systems like, for example, Sf9 of Hi5 cells, which can be infected with, for example, recombinant virus expression vectors (for example, baculovirus) containing the polynucleotide molecules of the invention; Xenopus oocytes, which can be injected with, for example, plasmids; plant cell systems, which can be infected with, for example, recombinant virus expression vectors (for example, cauliflower mosaic virus (CaMV) or tobacco mosaic virus (TMV)) or transformed with recombinant plasmid expression vectors (for example, Ti plasmid) containing a FcR or variant nucleotide sequence; or mammalian cell systems (for example, COS, CHO, BHK, HEK293, VERO, HeLa, MDCK, Wi38, Swiss 3T3 and NIH 3T3 cells), which can be transformed with recombinant expression constructs containing, for example, promoters derived, for example, from the genome of mammalian cells (for example, the metallothionein promoter) from mammalian viruses (for example, the adenovirus late promoter, CMV IE and the vaccinia virus 7.5K promoter) or from bacterial cells (for example, the tet-repressor binding is employed in the tet-on and tet-off systems). Also useful as host cells are primary or secondary cells obtained directly from a mammal and transfected with a plasmid vector or infected with a viral vector. Depending on the host cell and the respective vector used to introduce the polynucleotide of the invention the polynucleotide can integrate, for example, into the chromosome or the mitochondrial DNA or can be maintained extrachromosomally like, for example, episomally or can be only transiently comprised in the cells.
In the sequence of FcyRIIb three potential N-glycosylation sites are found. All three sites are on the surface of the molecule and are accessible. They are located in the EIF loops (N61 and N142) of both domains and on strand E (N 135) of the C-terminal domain. FcRs isolated from mammalian cells are highly glycosylated. Since FcR is glycosylated *in vivo* it may be desirable to chose an expression system, which provides faithful glycosylation of the protein. Consequently, it is preferred to introduce the polynucleotides encoding the FcR of the present invention into higher eukaryotic cells, in particular into mammalian cells, e.g. COS, CHO, BHK, HEK293, VERO, HeLa, MDCK, Wi38, Swiss 3T3 or NIH 3T3 cells.

Preferably the Fcγ receptor according to the invention lacks the transmebrane domain and/or the signal peptide and is soluble. Soluble forms of Fc receptors such as FcγRIII mediate isotype-specific regulation of B cell growth and immunoglobulin production. In a murine model of myeloma, sFcR suppresses growth and immunoglobulin production of tumor cells (Muller, *et al.,* 1985; Roman, *et al*., 1988; Teillaud, *et al.,* 1990). Furthermore, sFcR binds to surface IgG on cultures of human IgG-secreting myeloma cells and effects suppression of tumor cell growth and IgG secretion. Prolonged exposure of these cells to sFcR results in tumor cell cytolysis (Hoover, *et al.,* 1995).

The Fcγ receptor polypeptides can be any of those described above but with not more than ten (e.g., not more than: ten, nine, eight, seven, six, five, four, three, two, or one) conservative substitutions. Conservative substitutions are known in the art and typically include substitution of, e.g. one polar amino acid with another polar amino acid and one acidic amino acid with another acidic amino acid. Accordingly, conservative substitutions preferably include substitutions within the following groups of amino acids: glycine, alanine, valine, proline, isoleucine, and leucine (non polar, aliphatic side chain); aspartic acid and glutamic acid (negatively charged side chain); asparagine, glutamine, methionine, cysteine, serine and threonine (polar uncharged side chain); lysine, histidine and arginine; and phenylalanine, tryptophane and tyrosine (aromatic side chain); and lysine, arginine an histidine (positively charged side chain). It is well known in the art how to determine the effect of a given substitution, e.g. on pKI etc. All that is required of a polypeptide having one or more conservative substitutions is that it has at least 50% (e.g., at least: 55%; 60%; 65%, 70%; 75%; 80%; 85%; 90%; 95%; 98%; 99%; 99.5%; or 100% or more) of the activity of the unaltered Fcγ receptor according to the invention.

Both polypeptides and peptides can be produced by standard *in vitro* recombinant DNA techniques and *in vivo* transgenesis, using nucleotide sequences encoding the appropriate polypeptides or peptides. Methods well-known to those skilled in the art can be used to construct expression vectors containing relevant coding sequences and appropriate transcriptional/translational control signals. See, for example, the techniques described in Sambrook, et al., Molecular Cloning: A Laboratory Manual (2nd Ed.) [Cold Spring Harbor Laboratory, N.Y., 1989], and Ausubel, et al., Current Protocols in Molecular Biology [Green Publishing Associates and Wiley Interscience, N.Y., 1989].

Polypeptides and fragments of the invention, *i.e*. isolated polypeptides, also include those described above, but modified for *in vivo* use by the addition, at the amino- and/or carboxyl-terminal ends, of blocking agents to facilitate survival of the relevant polypeptide in vivo. This can be useful in those situations in which the peptide termini tend to be degraded by proteases prior to cellular up-take. Such blocking agents can include, without limitation, additional related or unrelated peptide sequences that can be attached to the amino and/or carboxyl terminal residues of the peptide to be administered. This can be done either chemically during the synthesis of the peptide or by recombinant DNA technology by methods familiar to artisans of average skill.

Alternatively, blocking agents such as pyroglutamic acid or other molecules known in the art can be attached to the amino and/or carboxyl terminal residues, or the amino group at the amino terminus or carboxyl group at the carboxyl terminus can be replaced with a different moiety. Likewise, the peptides can be covalently or noncovalently coupled to pharmaceutically acceptable "carrier" proteins prior to administration.

The term "isolated" polypeptide or peptide fragment as used herein refers to a polypeptide or a peptide fragment which either has no naturally-occurring counterpart or has been separated or purified from components which naturally accompany it, e.g., in tissues such as tongue, pancreas, liver, spleen, ovary, testis, muscle, joint tissue, neural tissue, gastrointestinal tissue or tumor tissue, or body fluids such as blood, serum, or urine. Typically, the polypeptide or peptide fragment is considered "isolated" when it is at least 70%, by dry weight, free from the proteins and other naturally-occurring organic molecules with which it is naturally associated. Preferably, a preparation of a polypeptide (or peptide fragment thereof) of the invention is at least 80%, more preferably at least 90%, and most preferably at least 99%, by dry weight, the polypeptide (or the peptide fragment thereof), respectively, of the invention. Thus, for example, a preparation of polypeptide x is at least 80%, more preferably at least 90%, and most preferably at least 99%, by dry weight, polypeptide x. Since a polypeptide that is chemically synthesized is, by its nature, separated from the components that naturally accompany it, the synthetic polypeptide is "isolated."

An isolated polypeptide (or peptide fragment) of the invention can be obtained, for example, by extraction from a natural source (e.g., from tissues or bodily fluids); by expression of a recombinant nucleic acid encoding the polypeptide; or by chemical synthesis. A polypeptide that is produced in a cellular system different from the source from which it naturally originates is "isolated," because it will necessarily be free of components which naturally accompany it. The degree of isolation or purity can be measured by any appropriate method, e.g., column chromatography, polyacrylamide gel electrophoresis, or HPLC analysis.

The Fcγ receptor according to the invention may be chemically modified (improved) by PEGylation and/or genetic engineering.

Known approaches involve the provision of additional glycosylation sites (see e.g. WO 91/05867, WO 94/09257 and WO 01/81405). Such modified analogs may have at least one additional N-linked and/or O-linked carbohydrate chain. Other attempts to improve the half life may involve the addition of polyethylene glycol residues (PEG) of varying length to the amino acid backbone (see e.g. WO 00/32772, WO 01/02017, WO 03/029291). One may modify the molecules with at least one N-linked and/or O-linked oligosaccharide which are further modified by oxidation, sulfation, phosphorylation PEGylation or a combination thereof (see WO 2005/025606). All these approaches can equally be employed to extend the half life of the variants of the present invention and accordingly in a preferred embodiment of the Fcγ receptor according to the invention the modification is selected from the group consisting of oxidation, sulfation, phosphorylation, addition of oligosaccharides or combinations thereof. If the addition of further N-linked or O-linked oligonucleotides is desired it is possible to introduce them by introducing additional glycosylation sites. It also preferred that the protein is affinity modulated.

In the practice of one aspect of the present invention, a pharmaceutical composition comprising the receptor of the invention may be administered to a mammal by any route which provides a sufficient level of activity. It can be administered systemically or locally. Such administration may be parenterally, transmucosally, e.g., orally, nasally, rectally, intravaginally, sub-lingually, submucosally or transdermally. Preferably, administration is parenteral, e.g., via intravenous or intraperitoneal injection, and also including, but is not limited to, intraarterial, intramuscular, intradermal and subcutaneous administration. If the pharmaceutical composition of the present invention is administered locally it can be injected directly into the organ or tissue to be treated. In cases of treating the nervous system this administration route includes, but is not limited to, the intracerebral, intraventricular, intracerebroventricular, intrathecal, intracistemal, intraspinal and/or peri-spinal routes of administration, which can employ intracranial and intravertebral needles, and catheters with or without pump devices.

The receptor may also by glycosylated.

Most preferably the receptor is selected from the group of FcγRIIB/C (CD32) and FcγRIIIA/B (CD16b). The invention also relates to isoforms thereof and isoforms of the FcRs claimed herein in general.

In a preferred embodiment of pharmaceutical composition comprises an FcR receptor polypeptide in a dosage unit form for treating multiple sclerosis, wherein the multiple sclerosis is a B cell mediated form of multiple sclerosis and/or an autoantibody driven form of multiple sclerosis, and the amount to administered to a patient in a single dose is between 1 and 20 mg/kg, preferably 2 and 10 mg/kg, more preferably between 25 and 5 mg/kg.

A pharmaceutical composition may additionally comprise on or more of the following substances, a detergent and/or a sugar. A preferred detergent is Tween 20. A preferred sugar is manitol.

The Fcγ receptor according to the invention preferably has sequence selected from the group of, SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12 and SEQ ID NO. 13. These are outlined in Table 1.

The Fcγ receptor according to the invention preferably comprises only the extra cellular domain of said sequences (soluble form of the receptor). Said domain is known from sequence alignments, is structurally characterized by x-ray crystallography and comprise the first two (CD16, CD32) or the first three (CD64) IgG-like domains of the mature receptor (Sondermann P., Kaiser J., Jacob U., Molecular basis for immune complex recognition: a comparison of Fc-receptor structures. J. Mol. Biol. 2001, 309, 737-749).

**Table 1**

| | | |
|---|---|---|
| Human FCgamma RIA (SEQ ID NO. 1) | MWFLTTLLLWVPVDGQVDTTKAVIT LQPPWVSVFQEETVTLHCEVLHLPG SSSTQWFLNGTATQTSTPSY RITSASVNDSGEYRCQRGLSGRSDP IQLEIHRGWLLLQVSSRVFTEGEPL ALRCHAWKDKLVYNVLYYRN GKAFKFFHWNSNLTILKTNISHNGT YHCSGMGKHRYTSAGISVTVKELFP APVLNASVTSPLLEGNLVTL SCETKLLLQRPGLQLYFSFYMGSKT LRGRNTSSEYQILTARREDSGLYWC EAATEDGNVLKRSPELELQV LGLQLPTPVWFHVLFYLAVGIMFLV NTVLWVTIRKELKRKKKWDLEISLD SGHEKKVISSLQEDRHLEEE LKCQEQKEEQLQEGVHRKEPQGAT | SEQ ID NO. 14 ATGTGGTTCTTGACAACTCTGCTCCTTTGGGTTCCAGT TGATGGGCAAGTGGACACCACAAAGGCAGTGATCACTT TGCAGCCTCCATGGGTCAGCGTGTTCCAAGAGGAAACC GTAACCTTGCACTGTGAGGTGCTCCATCTGCCTGGGAG CAGCTCTACACAGTGGTTTCTCAATGGCACAGCCACTC AGACCTCGACCCCCAGCTACAGAATCACCTCTGCCAGT GTCAATGACAGTGGTGAATACAGGTGCCAGAGAGGTCT CTCAGGGCGAAGTGACCCCATACAGCTGGAAATCCACA GAGGCTGGCTACTACTGCAGGTCTCCAGCAGAGTCTTC ACGGAAGGAGAACCTCTGGCCTTGAGGTGTCATGCGTG GAAGGATAAGCTGGTGTACAATGTGCTTTACTATCGAA ATGGCAAAGCCTTTAAGTTTTTCCACTGGAATTCTAAC CTCACCATTCTGAAAACCAACATAAGTCACAATGGCAC CTACCATTGCTCAGGCATGGGAAAGCATCGCTACACAT CAGCAGGAATATCTGTCACTGTGAAAGAGCTATTTCCA GCTCCAGTGCTGAATGCATCTGTGACATCCCCACTCCT GGAGGGGAATCTGGTCACCCTGAGCTGTGAAACAAAGT TGCTCTTGCAGAGGCCTGGTTTGCAGCTTTACTTCTCC TTCTACATGGGCAGCAAGACCCTGCGAGGCAGGAACAC ATCCTCTGAATACCAAATACTAACTGCTAGAAGAGAAG ACTCTGGGTTATACTGGTGCGAGGCTGCCACAGAGGAT GGAAATGTCCTTAAGCGCAGCCCTGAGTTGGAGCTTCA AGTGCTTGGCCTCCAGTTACCAACTCCTGTCTGGTTTC ATGTCCTTTTCTATCTGGCAGTGGGAATAATGTTTT TAGTGAACACTGTTCTCTGGGTGACAATACGTAAAGAA CTGAAAAGAAAGAAAAAGTGGGATTTAGAAATCTCTTT GGATTCTGGTCATGAGAAGAAGGTAATTTCCAGCCTTC AAGAAGACAGACATTTAGAAGAAGAGCTGAAATGTCAG GAACAAAAAGAAGAACAGCTGCAGGAAGGGGTGCACCG GAAGGAGCCCCAGGGGGCCACGTAG |
| Human FCgamma RIB (SEQ ID NO. 2) | MWFLTTLLLWGWLLLQVSSRVFMEG EPLALRCHAWKDKLVYNVLYYRNGK AFKFFHWNSNLTILKTNISH NGTYHCSGMGKHRYTSAGISQYTVK GLQLPTPVWFHVLFYLAVGIMFLVN TVLWVTIRKELKRKKKWNLE ISLDSGHEKKVISSLQEDRHLEEEL KCQEQKEEQLQEGVHRKEPQGAT | SEQ ID NO.15 ATGTGGTTCTTGACAACTCTGCTCCTTTGGGGCTGGCT ACTACTGCAGGTCTCCAGCAGAGTCTTCATGGAAGGAG AACCTCTGGCCTTGAGGTGTCATGCGTGGAAGGATAAG CTGGTGTACAATGTGCTTTACTATCGAAATGGCAAAGC CTTTAAGTTTTTCCACTGGAATTCTAACCTCACCATTC TGAAAACCAACATAAGTCACAATGGCACCTACCATTGC TCAGGCATGGGAAAGCATCGCTACACATCAGCAGGAAT ATCACAATACACTGTGAAAGGCCTCCAGTTACCAACTC CTGTCTGGTTTCATGTCCTTTTCTATCTGGCAGTGGGA ATAATGTTTTTAGTGAACACTGTTCTCTGGGTGACAAT ACGTAAAGAACTGAAAAGAAAGAAAAAGTGGAATTTAG AAATCTCTTTGGATTCTGGTCATGAGAAGAAGGTAATT TCCAGCCTTCAAGAAGACAGACATTTAGAAGAAGAGCT GAAATGTCAGGAACAAAAAGAAGAACAGCTGCAGGAAG GGGTGCACCGGAAGGAGCCCCAGGGGGCCACGTAG |
| Human FCgamma RIB (SEQ ID NO. 3) | MWFLTTLLLWVPVDGQVDTTKAVIT LQPPWVSVFQEETVTLHCEVLHLPG SSSTQWFLNGTATQTSTPSY RITSASVNDSGEYRCQRGLSGRSDP IQLEIHRGWLLLQVSSRVFMEGEPL ALRCHAWKDKLVYNVLYYRN GKAFKFFHWNSNLTILKTNISHNGT YHCSGMGKHRYTSAGISQYTVKGLQ LPTPVWFHVLFYLAVGIMFL VNTVLWVTIRKELKRKKKWNLEISL DSGHEKKVISSLQEDRHLEEELKCQ EQKEEQLQEGVHRKEPQGAT | SEQ ID NO. 16 ATGTGGTTCTTGACAACTCTGCTCCTTTGGGTTCCAGT TGATGGGCAAGTGGACACCACAAAGGCAGTGATCACTT TGCAGCCTCCATGGGTCAGCGTGTTCCAAGAGGAAACC GTAACCTTGCACTGTGAGGTGCTCCATCTGCCTGGGAG CAGCTCCACACAGTGGTTTCTCAATGGCACAGCCACTC AGACCTCGACCCCCAGCTACAGAATCACCTCTGCCAGT GTCAATGACAGTGGTGAATACAGGTGCCAGAGAGGTCT CTCAGGGCGAAGTGACCCCATACAGCTGGAAATCCACA GAGGCTGGCTACTACTGCAGGTCTCCAGCAGAGTCTTC ATGGAAGGAGAACCTCTGGCCTTGAGGTGTCATGCGTG GAAGGATAAGCTGGTGTACAATGTGCTTTACTATCGAA ATGGCAAAGCCTTTAAGTTTTTCCACTGGAATTCTAAC CTCACCATTCTGAAAACCAACATAAGTCACAATGGCAC CTACCATTGCTCAGGCATGGGAAAGCATCGCTACACAT CAGCAGGAATATCACAATACACTGTGAAAGGCCTCCAG TTACCAACTCCTGTCTGGTTTCATGTCCTTTTCTATCT GGCAGTGGGAATAATGTTTTTAGTGAACACTGTTCTCT GGGTGACAATACGTAAAGAACTGAAAAGAAAGAAAAAG TGGAATTTAGAAATCTCTTTGGATTCTGGTCATGAGAA GAAGGTAATTTCCAGCCTTCAAGAAGACAGACATTTAG AAGAAGAGCTGAAATGTCAGGAACAAAAAGAAGAACAG CTGCAGGAAGGGGTGCACCGGAAGGAGCCCCAGGGGGC CACGTAG |
| Human FCgamma RIIa (SEQ ID NO. 4) | MTMETQMSQNVCPRNLWLLQPLTVL LLLASADSQAAPPKAVLKLEPPWIN VLQEDSVTLTCQGARSPESD SIQWFHNGNLIPTHTQPSYRFKANN NDSGEYTCQTGQTSLSDPVHLTVLS EWLVLQTPHLEFQEGETIML RCHSWKDKPLVKVTFFQNGKSQKFS HLDPTFSIPQANHSHSGDYHCTGNI GYTLFSSKPVTITVQVPSMG SSSPMGIIVAVVIATAVAAIVAAVV ALIYCRKKRISANSTDPVKAAQFEP PGRQMIAIRKRQLEETNNDY ETADGGYMTLNPRAPTDDDKNIYLT LPPNDHVNSNN | SEQ ID NO. 17 ATGACTATGGAGACCCAAATGTCTCAGAATGTATGTCC CAGAAACCTGTGGCTGCTTCAACCATTGACAGTTTTGC TGCTGCTGGCTTCTGCAGACAGTCAAGCTGCTCCCCCA AAGGCTGTGCTGAAACTTGAGCCCCCGTGGATCAACGT GCTCCAGGAGGACTCTGTGACTCTGACATGCCAGGGGG CTCGCAGCCCTGAGAGCGACTCCATTCAGTGGTTCCAC AATGGGAATCTCATTCCCACCCACACGCAGCCCAGCTA CAGGTTCAAGGCCAACAACAATGACAGCGGGGAGTACA CGTGCCAGACTGGCCAGACCAGCCTCAGCGACCCTGTG CATCTGACTGTGCTTTCCGAATGGCTGGTGCTCCAGAC CCCTCACCTGGAGTTCCAGGAGGGAGAAACCATCATGC TGAGGTGCCACAGCTGGAAGGACAAGCCTCTGGTCAAG GTCACATTCTTCCAGAATGGAAAATCCCAGAAATTCTC CCATTTGGATCCCACCTTCTCCATCCCACAAGCAAACC ACAGTCACAGTGGTGATTACCACTGCACAGGAAACATA GGCTACACGCTGTTCTCATCCAAGCCTGTGACCATCAC TGTCCAAGTGCCCAGCATGGGCAGCTCTTCACCAATGG GGATCATTGTGGCTGTGGTCATTGCGACTGCTGTAGCA GCCATTGTTGCTGCTGTAGTGGCCTTGATCTACTGCAG GAAAAAGCGGATTTCAGCCAATTCCACTGATCCTGTGA AGGCTGCCCAATTTGAGCCACCTGGACGTCAAATGATT GCCATCAGAAAGAGACAACTTGAAGAAACCAACAATGA CTATGAAACAGCTGACGGCGGCTACATGACTCTGAACC CCAGGGCACCTACTGACGATGATAAAAACATCTACCTG ACTCTTCCTCCCAACGACCATGTCAACAGTAATAACTA A |
| Human FGgamma RIIB Isoform 1 (SEQ ID NO. 5) | MGILSFLPVLATESDWADCKSPQPW GHMLLWTAVLFLAPVAGTPAAPPKA VLKLEPQWINVLQEDSVTLT CRGTHSPESDSIQWFHNGNLIPTHT QPSYRFKANNNDSGEYTCQTGQTSL SDPVHLTVLSEWLVLQTPHL EFQEGETIVLRCHSWKDKPLVKVTF FQNGKSKKFSRSDPNFSIPQANHSH SGDYHCTGNIGYTLYSSKPV TITVQAPSSSPMGIIVAVVTGIAVA AIVAAVVALIYCRKKRISALPGYPE CREMGETLPEKPANPTNPDE ADKVGAENTITYSLLMHPDALEEPD DQNRI | SEQ ID NO. 18 ATGGGAATCCTGTCATTCTTACCTGTCCTTGCCACTGA GAGTGACTGGGCTGACTGCAAGTCCCCCCAGCCTTGGG GTCATATGCTTCTGTGGACAGCTGTGCTATTCCTGGCT CCTGTTGCTGGGACACCTGCAGCTCCCCCAAAGGCTGT GCTGAAACTCGAGCCCCAGTGGATCAACGTGCTCCAGG AGGACTCTGTGACTCTGACATGCCGGGGGACTCACAGC CCTGAGAGCGACTCCATTCAGTGGTTCCACAATGGGAA TCTCATTCCCACCCACACGCAGCCCAGCTACAGGTTCA AGGCCAACAACAATGACAGCGGGGAGTACACGTGCCAG ACTGGCCAGACCAGCCTCAGCGACCCTGTGCATCTGAC TGTGCTTTCTGAGTGGCTGGTGCTCCAGACCCCTCACC TGGAGTTCCAGGAGGGAGAAACCATCGTGCTGAGGTGC CACAGCTGGAAGGACAAGCCTCTGGTCAAGGTCACATT CTTCCAGAATGGAAAATCCAAGAAATTTTCCCGTTCGG ATCCCAACTTCTCCATCCCACAAGCAAACCACAGTCAC AGTGGTGATTACCACTGCACAGGAAACATAGGCTACAC GCTGTACTCATCCAAGCCTGTGACCATCACTGTCCAAG CTCCCAGCTCTTCACCGATGGGGATCATTGTGGCTGTG GTCACTGGGATTGCTGTAGCGGCCATTGTTGCTGCTGT AGTGGCCTTGATCTACTGCAGGAAAAAGCGGATTTCAG CTCTCCCAGGATACCCTGAGTGCAGGGAAATGGGAGAG ACCCTCCCTGAGAAACCAGCCAATCCCACTAATCCTGA TGAGGCTGACAAAGTTGGGGCTGAGAACACAATCACCT ATTCACTTCTCATGCACCCGGATGCTCTGGAAGAGCCT GATGACCAGAACCGTATTTAG |
| Human FCgamma RIIB Isoform 2 (SEQ ID NO. 6) | MGILSFLPVLATESDWADCKSPQPW GHMLLWTAVLFLAPVAGTPAPPKAV LKLEPQWINVLQEDSVTLTC RGTHSPESDSIQWFHNGNLIPTHTQ PSYRFKANNNDSGEYTCQTGQTSLS DPVHLTVLSEWLVLQTPHLE FQEGETIVLRCHSWKDKPLVKVTFF QNGKSKKFSRSDPNFSIPQANHSHS GDYHCTGNIGYTLYSSKPVT ITVQAPSSSPMGIIVAVVTGIAVAA IVAAVVALIYCRKKRISANPTNPDE ADKVGAENTITYSLLMHPDA LEEPDDQNRI | SEQ ID NO. 19 tctagcgagg tgacagcgta gaaccagaga atttgtttgc cctctagggt agaatccgcc aagctttgag agaaggctgt gactgctgtg ctctgggcgc cagctcgctc cagggagtgg tgggaatcct gtcattctta cctgtccttg ccactgagag tgactgggct gactgcaagt ccccccagcc ttggggtcat atgcttctgt ggacagctgt gctattcctg gctcctgttg ctgggacacc tgcagctccc ccaaaggctg tgctgaaact cgagccccag tggatcaacg tgctccagga ggactctgtg actctgacat gccgggggac tcacagccct gagagcgact ccattcagtg gttccacaat gggaatctca ttcccaccca cacgcagccc agctacaggt tcaaggccaa caacaatgac agcggggagt acacgtgcca gactggccag accagcctca gcgaccctgt gcatctgact gtgctttctg agtggctggt gctccagacc cctcacctgg agttccagga gggagaaacc atcgtgctga ggtgccacag ctggaaagac aagcctctgg tcaaggtcac attcttccag aatggaaaat ccaagaaatt ttcccgttcg gatcccaact tctccatccc acaagcaaac cacagtccca gtgggtgatt accactgcac aggaaaaaat agggctacac cctgtactca tccaag[?]cct gtggaccatc actgttcaaa gctccccaac ttcttcacgg atgggga |
| Human FCgamma RIIB Isoform 3 (SEQ ID NO. 7) | MGILSFLPVLATESDWADCKSPQPW GHMLLWTAVLFLAPVAGTPAAPPKA VLKLEPQWINVLQEDSVTLT CRGTHSPESDSIQWFHNGNLIPTHT QPSYRFKANNNDSGEYTCQTGQTSL SDPVHLTVLSEWLVLQTPHL EFQEGETIVLRCHSWKDKPLVKVTF FQNGKSKKFSRSDPNFSIPQANHSH SGDYHCTGNIGYTLYSSKPV TITVQAPSSSPMGIIVAVVTGIAVA AIVAAVVALIYCRKKRISANPTNPD EADKVGAENTITYSLLMHPD ALEEPDDQNRI | SEQ ID NO. 20 ATGGGAATCCTGTCATTCTTACCTGTCCTTGCCACTGA GAGTGACTGGGCTGACTGCAAGTCCCCCCAGCCTTGGG GTCATATGCTTCTGTGGACAGCTGTGCTATTCCTGGCT CCTGTTGCTGGGACACCTGCAGCTCCCCCAAAGGCTGT GCTGAAACTCGAGCCCCAGTGGATCAACGTGCTCCAGG AGGACTCTGTGACTCTGACATGCCGGGGGACTCACAGC CCTGAGAGCGACTCCATTCAGTGGTTCCACAATGGGAA TCTCATTCCCACCCACACGCAGCCCAGCTACAGGTTCA AGGCCAACAACAATGACAGCGGGGAGTACACGTGCCAG ACTGGCCAGACCAGCCTCAGCGACCCTGTGCATCTGAC TGTGCTTTCTGAGTGGCTGGTGCTCCAGACCCCTCACC TGGAGTTCCAGGAGGGAGAAACCATCGTGCTGAGGTGC CACAGCTGGAAGGACAAGCCTCTGGTCAAGGTCACATT CTTCCAGAATGGAAAATCCAAGAAATTTTCCCGTTCGG ATCCCAACTTCTCCATCCCACAAGCAAACCACAGTCAC AGTGGTGATTACCACTGCACAGGAAACATAGGCTACAC GCTGTACTCATCCAAGCCTGTGACCATCACTGTCCAAG CTCCCAGCTCTTCACCGATGGGGATCATTGTGGCTGTG GTCACTGGGATTGCTGTAGCGGCCATTGTTGCTGCTGT AGTGGCCTTGATCTACTGCAGGAAAAAGCGGATTTCAG CCAATCCCACTAATCCTGATGAGGCTGACAAAGTTGGG GCTGAGAACACAATCACCTATTCACTTCTCATGCACCC GGATGCTCTGGAAGAGCCTGATGACCAGAACCGTATTT AG |
| Human FCgamma RIIb Isoform 4 (SEQ ID NO. 8) | mgilsflpvl atesdwadck spqpwghmll wtavlflapv agtpappkav lklepqwinv lqedsvtltc rgthspesds iqwfhngnli pthtqpsyrf kannndsgey tcqtgqtsls dpvhltvlse wlvlqtphle fqegetivlr chswkdkplv kvtffqngks kkfsrsdpnf sipqanhshs gdyhctgnig ytlysskpvt itvqapsssp mgiivavvtg iavaaivaav valiycrkkr isalpgypec remgetlpek panptnpdea dkvgaentit ysllmhpdal eepddqnri | SEQ ID NO. 21 atgggaatcc tgtcattctt acctgtcctt gccactgaga gtgactgggc tgactgcaag tccccccagc cttggggtca tatgcttctg tggacagctg tgctattcct ggctcctgtt gctgggacac ctgctccccc aaaggctgtg ctgaaactcg agccccagtg gatcaacgtg ctccaggagg actctgtgac tctgacatgc cgggggactc acagccctga gagcgactcc attcagtggt tccacaatgg gaatctcatt cccacccaca cgcagcccag ctacaggttc aaggccaaca acaatgacag cggggagtac acgtgccaga ctggccagac cagcctcagc gaccctgtgc atctgactgt gctttctgag tggctggtgc tccagacccc tcacctggag ttccaggagg gagaaaccat cgtgctgagg tgccacagct ggaaggacaa gcctctggtc aaggtcacat tcttccagaa tggaaaatcc aagaaatttt cccgttcgga tcccaacttc tccatcccac aagcaaacca cagtcacagt ggtgattacc actgcacagg aaacataggc tacacgctgt actcatccaa gcctgtgacc atcactgtcc aagctcccag ctcttcaccg atggggatca ttgtggctgt ggtcactggg attgctgtag cggccattgt tgctgctgta gtggccttga tctactgcag gaaaaagcgg atttcagctc tcccaggata ccctgagtgc agggaaatgg gagagaccct ccctgagaaa ccagccaatc ccactaatcc tgatgaggct gacaaagttg gggctgagaa cacaatcacc tattcacttc tcatgcaccc ggatgctctg gaagagcctg atgaccagaa ccgtatttag |
| Human FCgamma RIIc Isoform 1 (SEQ ID NO. 9) | MGILSFLPVLATESDWADCKSPQPW GHMLLWTAVLFLAPVAGTP AAPPKAVLKLEPQWINVLQEDSVTL TCRGTHSPESDSIPWFHNGNLIPTH TQPSYRFK ANNNDSGEYTCQTGQTSLSDPVHLT VLSEWLVLQTPHLEFQEGETIVLRC HSWKDKPL VKVTFFQNGKSKKFSRSDPNFSIPQ ANHSHSGDYHCTGNIGYTLYSSKPV TITVQAPS SSPMGIIVAVVTGIAVAAIVAAVVA LIYCRKKRISANSTDPVKAAQFEPP GRQMIAIR KRQPEETNNDYETADGGYMTLNPRA PTDDDKNIYLTLPPNDHVNSNN | SEQ ID NO. 22 atgggaatcc tgtcattctt acctgtcctt gccactgaga gtgactgggc tgactgcaag tccccccagc cttggggtca tatgcttctg tggacagctg tgctattcct ggctcctgtt gctgggacac ctgcagctcc cccaaaggct gtgctgaaac tcgagcccca gtggatcaac gtgctccaag aggactctgt gactctgaca tgccggggga ctcacagccc tgagagcgac tccattccgt ggttccacaa tgggaatctc attcccaccc acacgcagcc cagctacagg ttcaaggcca acaacaatga cagcggggag tacacgtgcc agactggcca gaccagcctc agcgaccctg tgcatctgac tgtgctttct gagtggctgg tgctccagac ccctcacctg gagttccagg agggagaaac catcgtgctg aggtgccaca gctggaagga caagcctctg gtcaaggtca cattcttcca gaatggaaaa tccaagaaat tttcccgttc ggatcccaac ttctccatcc cacaagcaaa ccacagtcac agtggtgatt accactgcac aggaaacata ggctacacgc tgtactcatc caagcctgtg accatcactg tccaagctcc cagctcttca ccgatgggga tcattgtggc tgtggtcact gggattgctg tagcggccat tgttgctgct gtagtggcct tgatctactg caggaaaaag cggatttcag ccaattccac tgatcctgtg aaggctgccc aatttgagcc acctggacgt caaatgattg ccatcagaaa gagacaacct gaagaaacca acaatgacta tgaaacagct gacggcggct acatgactct gaaccccagg gcacctactg acgatgataa aaacatctac ctgactcttc ctcccaacga ccatgtcaac agtaataact aa |
| Human FCgamma RIIC Isoform 2 (SEQ ID NO. 10) | MGILSFLPVLATESDWADCKSPQPW GHMLLWTAVLFLAPVAGTP AAPPKAVLKLEPQWINVLQEDSVTL TCRGTHSPESDSIPWFHNGNLIPTH TQPSYRFK ANNNDSGEYTCQTGQTSLSDPVHLT VLSEWLVLQTPHLEFQEGETIVLRC HSWKDKPL VKVTFFQNGKSKKFSRSDPNFSIPQ ANHSHSGDYHCTGNIGYTLYSSKPV TITVQAPS SSPMGIIVAVVTGIAVAAIVAAVVA LIYCRKKRISANSTDPVKAAQFEML SCTHLDVK | SEQ ID NO. 23 atgggaatcc tgtcattctt acctgtcctt gccactgaga gtgactgggc tgactgcaag tccccccagc cttggggtca tatgcttctg tggacagctg tgctattcct ggctcctgtt gctgggacac ctgcagctcc cccaaaggct gtgctgaaac tcgagcccca gtggatcaac gtgctccaag aggactctgt gactctgaca tgccggggga ctcacagccc tgagagcgac tccattccgt ggttccacaa tgggaatctc attcccaccc acacgcagcc cagctacagg ttcaaggcca acaacaatga cagcggggag tacacgtgcc agactggcca gaccagcctc agcgaccctg tgcatctgac tgtgctttct gagtggctgg tgctccagac ccctcacctg gagttccagg agggagaaac catcgtgctg aggtgccaca gctggaagga caagcctctg gtcaaggtca cattcttcca gaatggaaaa tccaagaaat tttcccgttc ggatcccaac ttctccatcc cacaagcaaa ccacagtcac agtggtgatt accactgcac aggaaacata ggctacacgc tgtactcatc caagcctgtg accatcactg tccaagctcc cagctcttca ccgatgggga tcattgtggc tgtggtcact gggattgctg tagcggccat tgttgctgct gtagtggcct tgatctactg caggaaaaag cggatttcag ccaattccac tgatcctgtg aaggctgccc aatttgagat gctttcctgc acccacctgg acgtcaaatg a |
| Human FCgamma RIIC Isoform 3 (SEQ ID NO. 11) | MGILSFLPVLATESDWADCKSPQPW GHMLLWTAVLFLAPVAGTP AAPPKAVLKLEPQWINVLQEDSVTL TCRGTHSPESDSIPWFHNGNLIPTH TQPSYRFK ANNNDSGEYTCQTGQTSLSDPVHLT VLSEWLVLQTPHLEFQEGETIVLRC HSWKDKPL VKVTFFQNGKSKKFSRSDPNFSIPQ ANHSHSGDYHCTGNIGYTLYSSKPV TITVQAPS SSPMGIIVAVVTGIAVAAIVAAVVA LIYCRKKRISATWTSNDCHQKETT | SEQ ID NO. 24 atgggaatcc tgtcattctt acctgtcctt gccactgaga gtgactgggc tgactgcaag tccccccagc cttggggtca tatgcttctg tggacagctg tgctattcct ggctcctgtt gctgggacac ctgcagctcc cccaaaggct gtgctgaaac tcgagcccca gtggatcaac gtgctccaag aggactctgt gactctgaca tgccggggga ctcacagccc tgagagcgac tccattccgt ggttccacaa tgggaatctc attcccaccc acacgcagcc cagctacagg ttcaaggcca acaacaatga cagcggggag tacacgtgcc agactggcca gaccagcctc agcgaccctg tgcatctgac tgtgctttct gagtggctgg tgctccagac ccctcacctg gagttccagg agggagaaac catcgtgctg aggtgccaca gctggaagga caagcctctg gtcaaggtca cattcttcca gaatggaaaa tccaagaaat tttcccgttc ggatcccaac ttctccatcc cacaagcaaa ccacagtcac agtggtgatt accactgcac aggaaacata ggctacacgc tgtactcatc caagcctgtg accatcactg tccaagctcc cagctcttca ccgatgggga tcattgtggc tgtggtcact gggattgctg tagcggccat tgttgctgct gtagtggcct tgatctactg caggaaaaag cggatttcag ccacctggac gtcaaatgat tgccatcaga aagagacaac ctga |
| Human FCgamma RIIIA (SEQ ID NO. 12) | MGGGAGERLFTSSCLVGLVPLGLRI SLVTCPLQCGIMWQLLLPTALLLLV SAGMRTEDLPKAVVFLEPQW YRVLEKDSVTLKCQGAYSPEDNSTQ WFHNESLISSQASSYFIDAATVDDS GEYRCQTNLSTLSDPVQLEV HIGWLLLQAPRWVFKEEDPIHLRCH SWKNTALHKVTYLQNGKGRKYFHHN SDFYIPKATLKDSGSYFCRG LFGSKNVSSETVNITITQGLAVSTI SSFFPPGYQVSFCLVMVLLFAVDTG LYFSVKTNIRSSTRDWKDHK FKWRKDPQDK | SEQ ID NO. 25 ATGGGTGGAGGGGCTGGGGAAAGGCTGTTTACTTCCTC CTGTCTAGTCGGTTTGGTCCCTTTAGGGCTCCGGATAT CTTTGGTGACTTGTCCACTCCAGTGTGGCATCATGTGG CAGCTGCTCCTCCCAACTGCTCTGCTACTTCTAGTTTC AGCTGGCATGCGGACTGAAGATCTCCCAAAGGCTGTGG TGTTCCTGGAGCCTCAATGGTACAGGGTGCTCGAGAAG GACAGTGTGACTCTGAAGTGCCAGGGAGCCTACTCCCC TGAGGACAATTCCACACAGTGGTTTCACAATGAGAGCC TCATCTCAAGCCAGGCCTCGAGCTACTTCATTGACGCT GCCACAGTCGACGACAGTGGAGAGTACAGGTGCCAGAC AAACCTCTCCACCCTCAGTGACCCGGTGCAGCTAGAAG TCCATATCGGCTGGCTGTTGCTCCAGGCCCCTCGGTGG GTGTTCAAGGAGGAAGACCCTATTCACCTGAGGTGTCA CAGCTGGAAGAACACTGCTCTGCATAAGGTCACATATT TACAGAATGGCAAAGGCAGGAAGTATTTTCATCATAAT TCTGACTTCTACATTCCAAAAGCCACACTCAAAGACAG CGGCTCCTACTTCTGCAGGGGGCTTTTTGGGAGTAAAA ATGTGTCTTCAGAGACTGTGAACATCACCATCACTCAA GGTTTGGCAGTGTCAACCATCTCATCATTCTTTCCACC TGGGTACCAAGTCTCTTTCTGCTTGGTGATGGTACTCC TTTTTGCAGTGGACACAGGACTATATTTCTCTGTGAAG ACAAACATTCGAAGCTCAACAAGAGACTGGAAGGACCA TAAATTTAAATGGAGAAAGGACCCTCAAGACAAATGA |
| Human FCgamma RIIIB (SEQ ID NO. 13) | MWQLLLPTALLLLVSAGMRTEDLPK AVVFLEPQWYSVLEKDSVTLKCQGA YSPEDNSTQWFHNENLISSQ ASSYFIDAATVNDSGEYRCQTNLST LSDPVQLEVHIGWLLLQAPRWVFKE EDPIHLRCHSWKNTALHKVT YLQNGKDRKYFHHNSDFHIPKATLK DSGSYFCRGLVGSKNVSSETVNITI TQGLAVSTISSFSPPGYQVS FCLVMVLLFAVDTGLYFSVKTNI | SEQ ID NO. 26 ATGTGGCAGCTGCTCCTCCCAACTGCTCTGCTACTTCT AGTTTCAGCTGGCATGCGGACTGAAGATCTCCCAAAGG CTGTGGTGTTCCTGGAGCCTCAATGGTACAGCGTGCTT GAGAAGGACAGTGTGACTCTGAAGTGCCAGGGAGCCTA CTCCCCTGAGGACAATTCCACACAGTGGTTTCACAATG AGAACCTCATCTCAAGCCAGGCCTCGAGCTACTTCATT GACGCTGCCACAGTCAACGACAGTGGAGAGTACAGGTG CCAGACAAACCTCTCCACCCTCAGTGACCCGGTGCAGC TAGAAGTCCATATCGGCTGGCTGTTGCTCCAGGCCCCT CGGTGGGTGTTCAAGGAGGAAGACCCTATTCACCTGAG GTGTCACAGCTGGAAGAACACTGCTCTGCATAAGGTCA CATATTTACAGAATGGCAAAGACAGGAAGTATTTTCAT CATAATTCTGACTTCCACATTCCAAAAGCCACACTCAA AGATAGCGGCTCCTACTTCTGCAGGGGGCTTGTTGGGA GTAAAAATGTGTCTTCAGAGACTGTGAACATCACCATC ACTCAAGGTTTGGCAGTGTCAACCATCTCATCATTCTC TCCACCTGGGTACCAAGTCTCTTTCTGCTTGGTGATGG TACTCCTTTTTGCAGTGGACACAGGACTATATTTCTCT GTGAAGACAAACATTTGA |

The preferred FcRs are encoded by the following nucleic acids:
Human FCgammaRIA (SEQ ID NO. 1) is encoded by the sequence according to SEQ ID NO. 14.
Human FCgammaRIB (SEQ ID NO. 2) is encoded by the sequence according to SEQ ID NO. 15.
Human FCgammaRIB (SEQ ID NO. 3) is encoded by the sequence according to SEQ ID NO. 16.
Human FCgammaRIIa (SEQ ID NO. 4) is encoded by the sequence according to SEQ ID NO. 17.
Human FCgammARIIB Isoform 1 (SEQ ID NO. 5) is encoded by the sequence according to SEQ ID NO. 18.
Human FCgammaRIIB Isoform 2 (SEQ ID NO. 6) is encode by the sequence according to SEQ ID NO. 19.
Human FCgammaRIIB Isoform 3 (SEQ ID NO. 7) is encoded by the sequence according to SEQ ID NO. 20.
Human FCgammaRIIb Isoform 4 (SEQ ID NO. 8) is encoded by the sequence according to SEQ ID NO. 21.
Human FCgammaRIIc Isoform 1 (SEQ ID NO. 9) is encoded by the sequence according to SEQ ID NO. 22.
Human FCgammaRIIC Isoform 2 (SEQ ID NO. 10) is encoded by the sequence according to SEQ ID NO. 23.
Human FCgammaRIIC Isoform 3 (SEQ ID NO. 11) is encoded by the sequence according to SEQ ID NO. 24.
Human FCgammaRIIIA (SEQ ID NO. 12) is encoded by the sequence according to SEQ ID NO. 25.
Human FCgammaRIIIB (SEQ ID NO. 13) is encoded by the sequence according to SEQ ID NO. 26.

It is preferred that the FcγR is a recombinant non-glycosylated human soluble FcγRIIb preferably selected from the group of SEQ ID NO. 5, SEQ ID NO. 6, SEQ ID NO. 7 and SEQ ID NO. 8.

### EXAMPLES

The biologically active compound, the recombinant human soluble FcγRIIb molecule, is produced in inclusion bodies by fermentation of genetically engineered E. coli.

sFcγRIIb is produced by fermentation in *E. coli* strain BL21 (DE3) which has been transformed with an optimized cDNA sequence for the expression of sFcγRIIb.

The sFcγRIIb contains 4 cysteins to form 2 intra-molecular disulfide bonds between positions Cys26-Cys68 and cys107-Cys151. The N-terminal sequence corresponds to the consensus sequence of the eukaryotic signal peptidase. The C-terminus was generated by introduction of a stop codon after the SER-SER-PRO motive. No further modifications.

The sFcγRIIb has a molecular mass of 19,688.9 at native and of 19,692.9 at reducing conditions.

An overview of the manufacturing process of the drug substance is given in Table 2:

**Table 2**

| |
|---|
| One vial of WCB |
| Fermentation of *E. coli* cell substrate |
| Harvest of *E. coli* cell substrate |
| Isolation of inclusion bodies |
| Refolding of sFcγRIIb from inclusion bodies |
| Purification of sFcγRIIb to yield bulk material |
| Storage of sFcγRIIb bulk at -80°C |

### FIGURE CAPTIONS

### Fig. 1

EAE was induced on day zero in 6-8 week old C57/BI6j female mice by subcutaneous injection of 100 µg rat MOG in complete Freund adjuvant (CFA). 250ng of pertussistoxin were given intraperitoneally on day 0 and day 2. The mice were treated intraperitoneally with 200 µg sFcR after first symptoms appeared on day 8, 11 and 14. The scoring scheme was according to the degree of paralysis: 0=no paralysis, 1=tail paralysis, 2=hind legs paralysis, 3=front legs paralysis, 4=complete paralysis, 5=death.

### Fig. 2

EAE was induced on day zero in 6-8 week old SJL female mice by subcutaneous injection of 100 µg rat MOG in complete Freund adjuvant (CFA). 250ng of pertussistoxin were given intraperitoneally on day 0 and day 2. The mice were treated intraperitoneally with 200 µg sFcR after first symptoms appeared on day 9, 12 and 15. The scoring scheme was according to the degree of paralysis: 0=no paralysis, 1=tail paralysis, 2=hind legs paralysis, 3=front legs paralysis, 4=complete paralysis, 5=death.

SEQUENCE LISTING

<110>  SuppreMol GmbH

<120>  SUBSTANCES AND METHODS FOR THE TREATMENT OF B CELL MEDIATED
       MULTIPLE SCLEROSIS

<130>  P1673 EP BLN

<160>  26

<170>  PatentIn version 3.3

<210>  1
<211>  374
<212>  PRT
<213>  Homo sapiens

<400>  1

Met Trp Phe Leu Thr Thr Leu Leu Leu Trp Val Pro Val Asp Gly Gln
1               5                   10                  15

Val Asp Thr Thr Lys Ala Val Ile Thr Leu Gln Pro Pro Trp Val Ser
            20                  25                  30

Val Phe Gln Glu Glu Thr Val Thr Leu His Cys Glu Val Leu His Leu
        35                  40                  45

Pro Gly Ser Ser Ser Thr Gln Trp Phe Leu Asn Gly Thr Ala Thr Gln
    50                  55                  60

Thr Ser Thr Pro Ser Tyr Arg Ile Thr Ser Ala Ser Val Asn Asp Ser
65                  70                  75                  80

Gly Glu Tyr Arg Cys Gln Arg Gly Leu Ser Gly Arg Ser Asp Pro Ile
                85                  90                  95

Gln Leu Glu Ile His Arg Gly Trp Leu Leu Leu Gln Val Ser Ser Arg
            100                 105                 110

Val Phe Thr Glu Gly Glu Pro Leu Ala Leu Arg Cys His Ala Trp Lys
        115                 120                 125

Asp Lys Leu Val Tyr Asn Val Leu Tyr Tyr Arg Asn Gly Lys Ala Phe
    130                 135                 140

Lys Phe Phe His Trp Asn Ser Asn Leu Thr Ile Leu Lys Thr Asn Ile
145                 150                 155                 160

Ser His Asn Gly Thr Tyr His Cys Ser Gly Met Gly Lys His Arg Tyr
                165                 170                 175

Thr Ser Ala Gly Ile Ser Val Thr Val Lys Glu Leu Phe Pro Ala Pro
            180                 185                 190

Val Leu Asn Ala Ser Val Thr Ser Pro Leu Leu Glu Gly Asn Leu Val
        195                 200                 205

Thr Leu Ser Cys Glu Thr Lys Leu Leu Leu Gln Arg Pro Gly Leu Gln
    210                 215                 220

Leu Tyr Phe Ser Phe Tyr Met Gly Ser Lys Thr Leu Arg Gly Arg Asn
225                 230                 235                 240

Thr Ser Ser Glu Tyr Gln Ile Leu Thr Ala Arg Arg Glu Asp Ser Gly
                245                 250                 255

Leu Tyr Trp Cys Glu Ala Ala Thr Glu Asp Gly Asn Val Leu Lys Arg
            260                 265                 270

Ser Pro Glu Leu Glu Leu Gln Val Leu Gly Leu Gln Leu Pro Thr Pro
        275                 280                 285

Val Trp Phe His Val Leu Phe Tyr Leu Ala Val Gly Ile Met Phe Leu
    290                 295                 300

Val Asn Thr Val Leu Trp Val Thr Ile Arg Lys Glu Leu Lys Arg Lys
305                 310                 315                 320

Lys Lys Trp Asp Leu Glu Ile Ser Leu Asp Ser Gly His Glu Lys Lys
                325                 330                 335

Val Ile Ser Ser Leu Gln Glu Asp Arg His Leu Glu Glu Glu Leu Lys
            340                 345                 350

Cys Gln Glu Gln Lys Glu Glu Gln Leu Gln Glu Gly Val His Arg Lys
        355                 360                 365

Glu Pro Gln Gly Ala Thr
    370

<210>  2
<211>  291
<212>  PRT
<213>  Homo sapiens

<400>  2

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Ala Pro Pro
        35                  40                  45

Lys Ala Val Leu Lys Leu Glu Pro Gln Trp Ile Asn Val Leu Gln Glu

50                  55                  60

Asp Ser Val Thr Leu Thr Cys Arg Gly Thr His Ser Pro Glu Ser Asp
65                  70                  75                  80

Ser Ile Gln Trp Phe His Asn Gly Asn Leu Ile Pro Thr His Thr Gln
                85                  90                  95

Pro Ser Tyr Arg Phe Lys Ala Asn Asn Asn Asp Ser Gly Glu Tyr Thr
            100                 105                 110

Cys Gln Thr Gly Gln Thr Ser Leu Ser Asp Pro Val His Leu Thr Val
        115                 120                 125

Leu Ser Glu Trp Leu Val Leu Gln Thr Pro His Leu Glu Phe Gln Glu
    130                 135                 140

Gly Glu Thr Ile Val Leu Arg Cys His Ser Trp Lys Asp Lys Pro Leu
145                 150                 155                 160

Val Lys Val Thr Phe Phe Gln Asn Gly Lys Ser Lys Lys Phe Ser Arg
                165                 170                 175

Ser Asp Pro Asn Phe Ser Ile Pro Gln Ala Asn His Ser His Ser Gly
            180                 185                 190

Asp Tyr His Cys Thr Gly Asn Ile Gly Tyr Thr Leu Tyr Ser Ser Lys
        195                 200                 205

Pro Val Thr Ile Thr Val Gln Ala Pro Ser Ser Ser Pro Met Gly Ile
    210                 215                 220

Ile Val Ala Val Val Thr Gly Ile Ala Val Ala Ala Ile Val Ala Ala
225                 230                 235                 240

Val Val Ala Leu Ile Tyr Cys Arg Lys Lys Arg Ile Ser Ala Asn Pro
                245                 250                 255

Thr Asn Pro Asp Glu Ala Asp Lys Val Gly Ala Glu Asn Thr Ile Thr
            260                 265                 270

Tyr Ser Leu Leu Met His Pro Asp Ala Leu Glu Glu Pro Asp Asp Gln
        275                 280                 285

Asn Arg Ile
    290

<210>  3
<211>  280
<212>  PRT
<213>  Homo sapiens

<400> 3

Met Trp Phe Leu Thr Thr Leu Leu Leu Trp Val Pro Val Asp Gly Gln
1               5                   10                  15

Val Asp Thr Thr Lys Ala Val Ile Thr Leu Gln Pro Pro Trp Val Ser
            20                  25                  30

Val Phe Gln Glu Glu Thr Val Thr Leu His Cys Glu Val Leu His Leu
        35                  40                  45

Pro Gly Ser Ser Ser Thr Gln Trp Phe Leu Asn Gly Thr Ala Thr Gln
    50                  55                  60

Thr Ser Thr Pro Ser Tyr Arg Ile Thr Ser Ala Ser Val Asn Asp Ser
65                  70                  75                  80

Gly Glu Tyr Arg Cys Gln Arg Gly Leu Ser Gly Arg Ser Asp Pro Ile
                85                  90                  95

Gln Leu Glu Ile His Arg Gly Trp Leu Leu Leu Gln Val Ser Ser Arg
            100                 105                 110

Val Phe Met Glu Gly Glu Pro Leu Ala Leu Arg Cys His Ala Trp Lys
        115                 120                 125

Asp Lys Leu Val Tyr Asn Val Leu Tyr Tyr Arg Asn Gly Lys Ala Phe
    130                 135                 140

Lys Phe Phe His Trp Asn Ser Asn Leu Thr Ile Leu Lys Thr Asn Ile
145                 150                 155                 160

Ser His Asn Gly Thr Tyr His Cys Ser Gly Met Gly Lys His Arg Tyr
                165                 170                 175

Thr Ser Ala Gly Ile Ser Gln Tyr Thr Val Lys Gly Leu Gln Leu Pro
            180                 185                 190

Thr Pro Val Trp Phe His Val Leu Phe Tyr Leu Ala Val Gly Ile Met
        195                 200                 205

Phe Leu Val Asn Thr Val Leu Trp Val Thr Ile Arg Lys Glu Leu Lys
    210                 215                 220

Arg Lys Lys Lys Trp Asn Leu Glu Ile Ser Leu Asp Ser Gly His Glu
225                 230                 235                 240

Lys Lys Val Ile Ser Ser Leu Gln Glu Asp Arg His Leu Glu Glu Glu
                245                 250                 255

Leu Lys Cys Gln Glu Gln Lys Glu Glu Gln Leu Gln Glu Gly Val His
            260                 265                 270

Arg Lys Glu Pro Gln Gly Ala Thr
        275                 280

<210>  4
<211>  316
<212>  PRT
<213>  Homo sapiens

<400>  4

Met Thr Met Glu Thr Gln Met Ser Gln Asn Val Cys Pro Arg Asn Leu
1               5                   10                  15

Trp Leu Leu Gln Pro Leu Thr Val Leu Leu Leu Leu Ala Ser Ala Asp
            20                  25                  30

Ser Gln Ala Ala Pro Pro Lys Ala Val Leu Lys Leu Glu Pro Pro Trp
        35                  40                  45

Ile Asn Val Leu Gln Glu Asp Ser Val Thr Leu Thr Cys Gln Gly Ala
    50                  55                  60

Arg Ser Pro Glu Ser Asp Ser Ile Gln Trp Phe His Asn Gly Asn Leu
65                  70                  75                  80

Ile Pro Thr His Thr Gln Pro Ser Tyr Arg Phe Lys Ala Asn Asn Asn
                85                  90                  95

Asp Ser Gly Glu Tyr Thr Cys Gln Thr Gly Gln Thr Ser Leu Ser Asp
            100                 105                 110

Pro Val His Leu Thr Val Leu Ser Glu Trp Leu Val Leu Gln Thr Pro
        115                 120                 125

His Leu Glu Phe Gln Glu Gly Glu Thr Ile Met Leu Arg Cys His Ser
    130                 135                 140

Trp Lys Asp Lys Pro Leu Val Lys Val Thr Phe Phe Gln Asn Gly Lys
145                 150                 155                 160

Ser Gln Lys Phe Ser His Leu Asp Pro Thr Phe Ser Ile Pro Gln Ala
                165                 170                 175

Asn His Ser His Ser Gly Asp Tyr His Cys Thr Gly Asn Ile Gly Tyr
            180                 185                 190

Thr Leu Phe Ser Ser Lys Pro Val Thr Ile Thr Val Gln Val Pro Ser
        195                 200                 205

Met Gly Ser Ser Ser Pro Met Gly Ile Ile Val Ala Val Val Ile Ala
    210                 215                 220

Thr Ala Val Ala Ala Ile Val Ala Ala Val Val Ala Leu Ile Tyr Cys
225                 230                 235                 240

Arg Lys Lys Arg Ile Ser Ala Asn Ser Thr Asp Pro Val Lys Ala Ala
                245                 250                 255

Gln Phe Glu Pro Pro Gly Arg Gln Met Ile Ala Ile Arg Lys Arg Gln
            260                 265                 270

Leu Glu Glu Thr Asn Asn Asp Tyr Glu Thr Ala Asp Gly Gly Tyr Met
        275                 280                 285

Thr Leu Asn Pro Arg Ala Pro Thr Asp Asp Asp Lys Asn Ile Tyr Leu
    290                 295                 300

Thr Leu Pro Pro Asn Asp His Val Asn Ser Asn Asn
305                 310                 315

<210>  5
<211>  310
<212>  PRT
<213>  Homo sapiens

<400>  5

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Ala Pro Pro
        35                  40                  45

Lys Ala Val Leu Lys Leu Glu Pro Gln Trp Ile Asn Val Leu Gln Glu
    50                  55                  60

Asp Ser Val Thr Leu Thr Cys Arg Gly Thr His Ser Pro Glu Ser Asp
65                  70                  75                  80

Ser Ile Gln Trp Phe His Asn Gly Asn Leu Ile Pro Thr His Thr Gln
                85                  90                  95

Pro Ser Tyr Arg Phe Lys Ala Asn Asn Asn Asp Ser Gly Glu Tyr Thr
            100                 105                 110

Cys Gln Thr Gly Gln Thr Ser Leu Ser Asp Pro Val His Leu Thr Val
        115                 120                 125

Leu Ser Glu Trp Leu Val Leu Gln Thr Pro His Leu Glu Phe Gln Glu
    130                 135                 140

Gly Glu Thr Ile Val Leu Arg Cys His Ser Trp Lys Asp Lys Pro Leu
145                 150                 155                 160

Val Lys Val Thr Phe Phe Gln Asn Gly Lys Ser Lys Lys Phe Ser Arg
                165                 170                 175

Ser Asp Pro Asn Phe Ser Ile Pro Gln Ala Asn His Ser His Ser Gly
            180                 185                 190

Asp Tyr His Cys Thr Gly Asn Ile Gly Tyr Thr Leu Tyr Ser Ser Lys
        195                 200                 205

Pro Val Thr Ile Thr Val Gln Ala Pro Ser Ser Ser Pro Met Gly Ile
    210                 215                 220

Ile Val Ala Val Val Thr Gly Ile Ala Val Ala Ala Ile Val Ala Ala
225                 230                 235                 240

Val Val Ala Leu Ile Tyr Cys Arg Lys Lys Arg Ile Ser Ala Leu Pro
                245                 250                 255

Gly Tyr Pro Glu Cys Arg Glu Met Gly Glu Thr Leu Pro Glu Lys Pro
            260                 265                 270

Ala Asn Pro Thr Asn Pro Asp Glu Ala Asp Lys Val Gly Ala Glu Asn
        275                 280                 285

Thr Ile Thr Tyr Ser Leu Leu Met His Pro Asp Ala Leu Glu Glu Pro
    290                 295                 300

Asp Asp Gln Asn Arg Ile
305                 310

<210>  6
<211>  290
<212>  PRT
<213>  Homo sapiens

<400>  6

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Pro Pro Lys
        35                  40                  45

Ala Val Leu Lys Leu Glu Pro Gln Trp Ile Asn Val Leu Gln Glu Asp
    50                  55                  60

Ser Val Thr Leu Thr Cys Arg Gly Thr His Ser Pro Glu Ser Asp Ser

65                  70                  75                  80

Ile Gln Trp Phe His Asn Gly Asn Leu Ile Pro Thr His Thr Gln Pro
                85                  90                  95

Ser Tyr Arg Phe Lys Ala Asn Asn Asn Asp Ser Gly Glu Tyr Thr Cys
            100                 105                 110

Gln Thr Gly Gln Thr Ser Leu Ser Asp Pro Val His Leu Thr Val Leu
        115                 120                 125

Ser Glu Trp Leu Val Leu Gln Thr Pro His Leu Glu Phe Gln Glu Gly
    130                 135                 140

Glu Thr Ile Val Leu Arg Cys His Ser Trp Lys Asp Lys Pro Leu Val
145                 150                 155                 160

Lys Val Thr Phe Phe Gln Asn Gly Lys Ser Lys Lys Phe Ser Arg Ser
                165                 170                 175

Asp Pro Asn Phe Ser Ile Pro Gln Ala Asn His Ser His Ser Gly Asp
            180                 185                 190

Tyr His Cys Thr Gly Asn Ile Gly Tyr Thr Leu Tyr Ser Ser Lys Pro
        195                 200                 205

Val Thr Ile Thr Val Gln Ala Pro Ser Ser Ser Pro Met Gly Ile Ile
    210                 215                 220

Val Ala Val Val Thr Gly Ile Ala Val Ala Ala Ile Val Ala Ala Val
225                 230                 235                 240

Val Ala Leu Ile Tyr Cys Arg Lys Lys Arg Ile Ser Ala Asn Pro Thr
                245                 250                 255

Asn Pro Asp Glu Ala Asp Lys Val Gly Ala Glu Asn Thr Ile Thr Tyr
            260                 265                 270

Ser Leu Leu Met His Pro Asp Ala Leu Glu Glu Pro Asp Asp Gln Asn
        275                 280                 285

Arg Ile
    290

<210>  7
<211>  291
<212>  PRT
<213>  Homo sapiens

<400>  7

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Ala Pro Pro
        35                  40                  45

Lys Ala Val Leu Lys Leu Glu Pro Gln Trp Ile Asn Val Leu Gln Glu
    50                  55                  60

Asp Ser Val Thr Leu Thr Cys Arg Gly Thr His Ser Pro Glu Ser Asp
65                  70                  75                  80

Ser Ile Gln Trp Phe His Asn Gly Asn Leu Ile Pro Thr His Thr Gln
                85                  90                  95

Pro Ser Tyr Arg Phe Lys Ala Asn Asn Asn Asp Ser Gly Glu Tyr Thr
            100                 105                 110

Cys Gln Thr Gly Gln Thr Ser Leu Ser Asp Pro Val His Leu Thr Val
        115                 120                 125

Leu Ser Glu Trp Leu Val Leu Gln Thr Pro His Leu Glu Phe Gln Glu
    130                 135                 140

Gly Glu Thr Ile Val Leu Arg Cys His Ser Trp Lys Asp Lys Pro Leu
145                 150                 155                 160

Val Lys Val Thr Phe Phe Gln Asn Gly Lys Ser Lys Lys Phe Ser Arg
                165                 170                 175

Ser Asp Pro Asn Phe Ser Ile Pro Gln Ala Asn His Ser His Ser Gly
            180                 185                 190

Asp Tyr His Cys Thr Gly Asn Ile Gly Tyr Thr Leu Tyr Ser Ser Lys
        195                 200                 205

Pro Val Thr Ile Thr Val Gln Ala Pro Ser Ser Ser Pro Met Gly Ile
    210                 215                 220

Ile Val Ala Val Val Thr Gly Ile Ala Val Ala Ala Ile Val Ala Ala
225                 230                 235                 240

Val Val Ala Leu Ile Tyr Cys Arg Lys Lys Arg Ile Ser Ala Asn Pro
                245                 250                 255

Thr Asn Pro Asp Glu Ala Asp Lys Val Gly Ala Glu Asn Thr Ile Thr
            260                 265                 270

Tyr Ser Leu Leu Met His Pro Asp Ala Leu Glu Glu Pro Asp Asp Gln
        275                 280                 285

Asn Arg Ile
    290

<210>  8
<211>  309
<212>  PRT
<213>  Homo sapiens

<400>  8

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Pro Pro Lys
        35                  40                  45

Ala Val Leu Lys Leu Glu Pro Gln Trp Ile Asn Val Leu Gln Glu Asp
    50                  55                  60

Ser Val Thr Leu Thr Cys Arg Gly Thr His Ser Pro Glu Ser Asp Ser
65                  70                  75                  80

Ile Gln Trp Phe His Asn Gly Asn Leu Ile Pro Thr His Thr Gln Pro
                85                  90                  95

Ser Tyr Arg Phe Lys Ala Asn Asn Asn Asp Ser Gly Glu Tyr Thr Cys
            100                 105                 110

Gln Thr Gly Gln Thr Ser Leu Ser Asp Pro Val His Leu Thr Val Leu
        115                 120                 125

Ser Glu Trp Leu Val Leu Gln Thr Pro His Leu Glu Phe Gln Glu Gly
    130                 135                 140

Glu Thr Ile Val Leu Arg Cys His Ser Trp Lys Asp Lys Pro Leu Val
145                 150                 155                 160

Lys Val Thr Phe Phe Gln Asn Gly Lys Ser Lys Lys Phe Ser Arg Ser
                165                 170                 175

Asp Pro Asn Phe Ser Ile Pro Gln Ala Asn His Ser His Ser Gly Asp
            180                 185                 190

Tyr His Cys Thr Gly Asn Ile Gly Tyr Thr Leu Tyr Ser Ser Lys Pro
        195                 200                 205

Val Thr Ile Thr Val Gln Ala Pro Ser Ser Ser Pro Met Gly Ile Ile
    210                 215                 220

Val Ala Val Val Thr Gly Ile Ala Val Ala Ala Ile Val Ala Ala Val
225                 230                 235                 240

Val Ala Leu Ile Tyr Cys Arg Lys Lys Arg Ile Ser Ala Leu Pro Gly
                245                 250                 255

Tyr Pro Glu Cys Arg Glu Met Gly Glu Thr Leu Pro Glu Lys Pro Ala
            260                 265                 270

Asn Pro Thr Asn Pro Asp Glu Ala Asp Lys Val Gly Ala Glu Asn Thr
        275                 280                 285

Ile Thr Tyr Ser Leu Leu Met His Pro Asp Ala Leu Glu Glu Pro Asp
    290                 295                 300

Asp Gln Asn Arg Ile
305

<210>  9
<211>  323
<212>  PRT
<213>  Homo sapiens

<400>  9

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Ala Pro Pro
        35                  40                  45

Lys Ala Val Leu Lys Leu Glu Pro Gln Trp Ile Asn Val Leu Gln Glu
    50                  55                  60

Asp Ser Val Thr Leu Thr Cys Arg Gly Thr His Ser Pro Glu Ser Asp
65                  70                  75                  80

Ser Ile Pro Trp Phe His Asn Gly Asn Leu Ile Pro Thr His Thr Gln
                85                  90                  95

Pro Ser Tyr Arg Phe Lys Ala Asn Asn Asn Asp Ser Gly Glu Tyr Thr
            100                 105                 110

Cys Gln Thr Gly Gln Thr Ser Leu Ser Asp Pro Val His Leu Thr Val
        115                 120                 125

Leu Ser Glu Trp Leu Val Leu Gln Thr Pro His Leu Glu Phe Gln Glu
    130                 135                 140

Gly Glu Thr Ile Val Leu Arg Cys His Ser Trp Lys Asp Lys Pro Leu
145                 150                 155                 160

Val Lys Val Thr Phe Phe Gln Asn Gly Lys Ser Lys Lys Phe Ser Arg
                165                 170                 175

Ser Asp Pro Asn Phe Ser Ile Pro Gln Ala Asn His Ser His Ser Gly
            180                 185                 190

Asp Tyr His Cys Thr Gly Asn Ile Gly Tyr Thr Leu Tyr Ser Ser Lys
        195                 200                 205

Pro Val Thr Ile Thr Val Gln Ala Pro Ser Ser Ser Pro Met Gly Ile
    210                 215                 220

Ile Val Ala Val Val Thr Gly Ile Ala Val Ala Ala Ile Val Ala Ala
225                 230                 235                 240

Val Val Ala Leu Ile Tyr Cys Arg Lys Lys Arg Ile Ser Ala Asn Ser
                245                 250                 255

Thr Asp Pro Val Lys Ala Ala Gln Phe Glu Pro Pro Gly Arg Gln Met
            260                 265                 270

Ile Ala Ile Arg Lys Arg Gln Pro Glu Glu Thr Asn Asn Asp Tyr Glu
        275                 280                 285

Thr Ala Asp Gly Gly Tyr Met Thr Leu Asn Pro Arg Ala Pro Thr Asp
    290                 295                 300

Asp Asp Lys Asn Ile Tyr Leu Thr Leu Pro Pro Asn Asp His Val Asn
305                 310                 315                 320

Ser Asn Asn

<210>  10
<211>  276
<212>  PRT
<213>  Homo sapiens

<400>  10

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Ala Pro Pro
        35                  40                  45

Lys Ala Val Leu Lys Leu Glu Pro Gln Trp Ile Asn Val Leu Gln Glu

50                  55                  60

Asp Ser Val Thr Leu Thr Cys Arg Gly Thr His Ser Pro Glu Ser Asp
65                  70                  75                  80

Ser Ile Pro Trp Phe His Asn Gly Asn Leu Ile Pro Thr His Thr Gln
                85                  90                  95

Pro Ser Tyr Arg Phe Lys Ala Asn Asn Asn Asp Ser Gly Glu Tyr Thr
            100                 105                 110

Cys Gln Thr Gly Gln Thr Ser Leu Ser Asp Pro Val His Leu Thr Val
        115                 120                 125

Leu Ser Glu Trp Leu Val Leu Gln Thr Pro His Leu Glu Phe Gln Glu
    130                 135                 140

Gly Glu Thr Ile Val Leu Arg Cys His Ser Trp Lys Asp Lys Pro Leu
145                 150                 155                 160

Val Lys Val Thr Phe Phe Gln Asn Gly Lys Ser Lys Lys Phe Ser Arg
                165                 170                 175

Ser Asp Pro Asn Phe Ser Ile Pro Gln Ala Asn His Ser His Ser Gly
            180                 185                 190

Asp Tyr His Cys Thr Gly Asn Ile Gly Tyr Thr Leu Tyr Ser Ser Lys
        195                 200                 205

Pro Val Thr Ile Thr Val Gln Ala Pro Ser Ser Ser Pro Met Gly Ile
    210                 215                 220

Ile Val Ala Val Val Thr Gly Ile Ala Val Ala Ala Ile Val Ala Ala
225                 230                 235                 240

Val Val Ala Leu Ile Tyr Cys Arg Lys Lys Arg Ile Ser Ala Asn Ser
                245                 250                 255

Thr Asp Pro Val Lys Ala Ala Gln Phe Glu Met Leu Ser Cys Thr His
            260                 265                 270

Leu Asp Val Lys
        275

<210>  11
<211>  267
<212>  PRT
<213>  Homo sapiens

<400>  11

Met Gly Ile Leu Ser Phe Leu Pro Val Leu Ala Thr Glu Ser Asp Trp
1               5                   10                  15

Ala Asp Cys Lys Ser Pro Gln Pro Trp Gly His Met Leu Leu Trp Thr
            20                  25                  30

Ala Val Leu Phe Leu Ala Pro Val Ala Gly Thr Pro Ala Ala Pro Pro
        35                  40                  45

Lys Ala Val Leu Lys Leu Glu Pro Gln Trp Ile Asn Val Leu Gln Glu
    50                  55                  60

Asp Ser Val Thr Leu Thr Cys Arg Gly Thr His Ser Pro Glu Ser Asp
65                  70                  75                  80

Ser Ile Pro Trp Phe His Asn Gly Asn Leu Ile Pro Thr His Thr Gln
                85                  90                  95

Pro Ser Tyr Arg Phe Lys Ala Asn Asn Asn Asp Ser Gly Glu Tyr Thr
            100                 105                 110

Cys Gln Thr Gly Gln Thr Ser Leu Ser Asp Pro Val His Leu Thr Val
        115                 120                 125

Leu Ser Glu Trp Leu Val Leu Gln Thr Pro His Leu Glu Phe Gln Glu
    130                 135                 140

Gly Glu Thr Ile Val Leu Arg Cys His Ser Trp Lys Asp Lys Pro Leu
145                 150                 155                 160

Val Lys Val Thr Phe Phe Gln Asn Gly Lys Ser Lys Lys Phe Ser Arg
                165                 170                 175

Ser Asp Pro Asn Phe Ser Ile Pro Gln Ala Asn His Ser His Ser Gly
            180                 185                 190

Asp Tyr His Cys Thr Gly Asn Ile Gly Tyr Thr Leu Tyr Ser Ser Lys
        195                 200                 205

Pro Val Thr Ile Thr Val Gln Ala Pro Ser Ser Ser Pro Met Gly Ile
    210                 215                 220

Ile Val Ala Val Val Thr Gly Ile Ala Val Ala Ala Ile Val Ala Ala
225                 230                 235                 240

Val Val Ala Leu Ile Tyr Cys Arg Lys Lys Arg Ile Ser Ala Thr Trp
                245                 250                 255

Thr Ser Asn Asp Cys His Gln Lys Glu Thr Thr
            260                 265

<210>  12
<211>  254

<212>  PRT
<213>  Homo sapiens

<400>  12

<210> 13
<211> 85
<212> PRT
<213> Homo sapiens

<400> 13

Asp Leu Pro Lys Ala Val Val Phe Leu Glu Pro Gln Trp Tyr Ser Val
1               5                   10                  15

Leu Glu Lys Asp Ser Val Thr Leu Lys Cys Gln Gly Ala Tyr Ser Pro
            20                  25                  30

Glu Asp Asn Ser Thr Gln Trp Phe His Asn Glu Ser Leu Ile Ser Ser
        35                  40                  45

Gln Ala Ser Ser Tyr Phe Ile Asp Ala Ala Thr Val Asn Asp Ser Gly
    50                  55                  60

Glu Tyr Arg Cys Gln Thr Asn Leu Ser Thr Leu Ser Asp Pro Val Gln
65                  70                  75                  80

Leu Glu Val His Ile
                85

<210> 14
<211> 1125
<212> DNA
<213> Homo sapiens

<400> 14
atgtggttct tgacaactct gctcctttgg gttccagttg atgggcaagt ggacaccaca 60

aaggcagtga tcactttgca gcctccatgg gtcagcgtgt tccaagagga aaccgtaacc 120

ttgcactgtg aggtgctcca tctgcctggg agcagctcta cacagtggtt tctcaatggc 180

acagccactc agacctcgac ccccagctac agaatcacct ctgccagtgt caatgacagt 240

ggtgaataca ggtgccagag aggtctctca gggcgaagtg accccataca gctggaaatc 300

cacagaggct ggctactact gcaggtctcc agcagagtct tcacggaagg agaacctctg 360

gccttgaggt gtcatgcgtg gaaggataag ctggtgtaca atgtgcttta ctatcgaaat 420

ggcaaagcct ttaagttttt ccactggaat tctaacctca ccattctgaa aaccaacata 480

agtcacaatg gcacctacca ttgctcaggc atgggaaagc atcgctacac atcagcagga 540

atatctgtca ctgtgaaaga gctatttcca gctccagtgc tgaatgcatc tgtgacatcc 600

ccactcctgg aggggaatct ggtcaccctg agctgtgaaa caaagttgct cttgcagagg 660

cctggtttgc agctttactt ctccttctac atgggcagca agaccctgcg aggcaggaac 720

acatcctctg aataccaaat actaactgct agaagagaag actctgggtt atactggtgc 780

gaggctgcca cagaggatgg aaatgtcctt aagcgcagcc ctgagttgga gcttcaagtg 840

cttggcctcc agttaccaac tcctgtctgg tttcatgtcc ttttctatct ggcagtggga 900

ataatgtttt tagtgaacac tgttctctgg gtgacaatac gtaaagaact gaaaagaaag 960

aaaaagtggg atttagaaat ctctttggat tctggtcatg agaagaaggt aatttccagc 1020

cttcaagaag acagacattt agaagaagag ctgaaatgtc aggaacaaaa agaagaacag 1080

ctgcaggaag gggtgcaccg gaaggagccc cagggggcca cgtag 1125

<210> 15
<211> 567
<212> DNA
<213> Homo sapiens

<400> 15
atgtggttct tgacaactct gctcctttgg ggctggctac tactgcaggt ctccagcaga 60

gtcttcatgg aaggagaacc tctggccttg aggtgtcatg cgtggaagga taagctggtg 120

tacaatgtgc tttactatcg aaatggcaaa gcctttaagt ttttccactg gaattctaac 180

ctcaccattc tgaaaaccaa cataagtcac aatggcacct accattgctc aggcatggga 240

aagcatcgct acacatcagc aggaatatca caatacactg tgaaaggcct ccagttacca 300

actcctgtct ggtttcatgt ccttttctat ctggcagtgg gaataatgtt tttagtgaac 360

actgttctct gggtgacaat acgtaaagaa ctgaaaagaa agaaaaagtg gaatttagaa 420

atctctttgg attctggtca tgagaagaag gtaatttcca gccttcaaga agacagacat 480

ttagaagaag agctgaaatg tcaggaacaa aaagaagaac agctgcagga aggggtgcac 540

cggaaggagc cccagggggc cacgtag 567

<210> 16
<211> 843
<212> DNA
<213> Homo sapiens

<400> 16
atgtggttct tgacaactct gctcctttgg gttccagttg atgggcaagt ggacaccaca 60

aaggcagtga tcactttgca gcctccatgg gtcagcgtgt tccaagagga aaccgtaacc 120

ttgcactgtg aggtgctcca tctgcctggg agcagctcca cacagtggtt tctcaatggc 180

acagccactc agacctcgac ccccagctac agaatcacct ctgccagtgt caatgacagt 240

ggtgaataca ggtgccagag aggtctctca gggcgaagtg accccataca gctggaaatc 300

cacagaggct ggctactact gcaggtctcc agcagagtct tcatggaagg agaacctctg 360

gccttgaggt gtcatgcgtg gaaggataag ctggtgtaca atgtgcttta ctatcgaaat 420

ggcaaagcct ttaagttttt ccactggaat tctaacctca ccattctgaa aaccaacata 480

agtcacaatg gcacctacca ttgctcaggc atgggaaagc atcgctacac atcagcagga 540

atatcacaat acactgtgaa aggcctccag ttaccaactc ctgtctggtt tcatgtcctt 600

ttctatctgg cagtgggaat aatgttttta gtgaacactg ttctctgggt gacaatacgt 660

aaagaactga aaagaaagaa aaagtggaat ttagaaatct ctttggattc tggtcatgag 720

aagaaggtaa tttccagcct tcaagaagac agacatttag aagaagagct gaaatgtcag 780

gaacaaaaag aagaacagct gcaggaaggg gtgcaccgga aggagcccca gggggccacg     840

tag                                                                  843

<210>  17
<211>  951
<212>  DNA
<213>  Homo sapiens

<400>  17
atgactatgg agacccaaat gtctcagaat gtatgtccca gaaacctgtg gctgcttcaa      60

ccattgacag ttttgctgct gctggcttct gcagacagtc aagctgctcc cccaaaggct     120

gtgctgaaac ttgagccccc gtggatcaac gtgctccagg aggactctgt gactctgaca     180

tgccaggggg ctcgcagccc tgagagcgac tccattcagt ggttccacaa tgggaatctc     240

attcccaccc acacgcagcc cagctacagg ttcaaggcca acaacaatga cagcggggag     300

tacacgtgcc agactggcca gaccagcctc agcgaccctg tgcatctgac tgtgctttcc     360

gaatggctgg tgctccagac ccctcacctg gagttccagg agggagaaac catcatgctg     420

aggtgccaca gctggaagga caagcctctg gtcaaggtca cattcttcca gaatggaaaa     480

tcccagaaat tctcccattt ggatcccacc ttctccatcc cacaagcaaa ccacagtcac     540

agtggtgatt accactgcac aggaaacata ggctacacgc tgttctcatc caagcctgtg     600

accatcactg tccaagtgcc cagcatgggc agctcttcac caatggggat cattgtggct     660

gtggtcattg cgactgctgt agcagccatt gttgctgctg tagtggcctt gatctactgc     720

aggaaaaagc ggatttcagc caattccact gatcctgtga aggctgccca atttgagcca     780

cctggacgtc aaatgattgc catcagaaag agacaacttg aagaaaccaa caatgactat     840

gaaacagctg acggcggcta catgactctg aaccccaggg cacctactga cgatgataaa     900

aacatctacc tgactcttcc tcccaacgac catgtcaaca gtaataacta a              951

<210>  18
<211>  933
<212>  DNA
<213>  Homo sapiens

<400>  18
atgggaatcc tgtcattctt acctgtcctt gccactgaga gtgactgggc tgactgcaag      60

tccccccagc cttggggtca tatgcttctg tggacagctg tgctattcct ggctcctgtt     120

gctgggacac ctgcagctcc cccaaaggct gtgctgaaac tcgagcccca gtggatcaac     180

gtgctccagg aggactctgt gactctgaca tgccggggga ctcacagccc tgagagcgac     240

tccattcagt ggttccacaa tgggaatctc attcccaccc acacgcagcc cagctacagg     300

ttcaaggcca acaacaatga cagcggggag tacacgtgcc agactggcca gaccagcctc     360

agcgaccctg tgcatctgac tgtgctttct gagtggctgg tgctccagac ccctcacctg     420

gagttccagg agggagaaac catcgtgctg aggtgccaca gctggaagga caagcctctg     480

gtcaaggtca cattcttcca gaatggaaaa tccaagaaat tttcccgttc ggatcccaac     540

ttctccatcc cacaagcaaa ccacagtcac agtggtgatt accactgcac aggaaacata 600

ggctacacgc tgtactcatc caagcctgtg accatcactg tccaagctcc cagctcttca 660

ccgatgggga tcattgtggc tgtggtcact gggattgctg tagcggccat tgttgctgct 720

gtagtggcct tgatctactg caggaaaaag cggatttcag ctctcccagg ataccctgag 780

tgcagggaaa tgggagagac cctccctgag aaaccagcca atcccactaa tcctgatgag 840

gctgacaaag ttggggctga gaacacaatc acctattcac ttctcatgca cccggatgct 900

ctggaagagc ctgatgacca gaaccgtatt tag 933

<210> 19
<211> 798
<212> DNA
<213> Homo sapiens

<400> 19
tctagcgagg tgacagcgta gaaccagaga atttgtttgc cctctagggt agaatccgcc 60

aagctttgag agaaggctgt gactgctgtg ctctgggcgc cagctcgctc cagggagtgg 120

tgggaatcct gtcattctta cctgtccttg ccactgagag tgactgggct gactgcaagt 180

ccccccagcc ttggggtcat atgcttctgt ggacagctgt gctattcctg gctcctgttg 240

ctgggacacc tgcagctccc ccaaaggctg tgctgaaact cgagccccag tggatcaacg 300

tgctccagga ggactctgtg actctgacat gccgggggac tcacagccct gagagcgact 360

ccattcagtg gttccacaat gggaatctca ttcccaccca cacgcagccc agctacaggt 420

tcaaggccaa caacaatgac agcggggagt acacgtgcca gactggccag accagcctca 480

gcgaccctgt gcatctgact gtgctttctg agtggctggt gctccagacc cctcacctgg 540

agttccagga gggagaaacc atcgtgctga ggtgccacag ctggaaagac aagcctctgg 600

tcaaggtcac attcttccag aatggaaaat ccaagaaatt ttcccgttcg gatcccaact 660

tctccatccc acaagcaaac cacagtccca gtgggtgatt accactgcac aggaaaaaat 720

agggctacac cctgtactca tccaagccct gtggaccatc actgttcaaa gctccccaac 780

ttcttcacgg atgggga 798

<210> 20
<211> 876
<212> DNA
<213> Homo sapiens

<400> 20
atgggaatcc tgtcattctt acctgtcctt gccactgaga gtgactgggc tgactgcaag 60

tccccccagc cttggggtca tatgcttctg tggacagctg tgctattcct ggctcctgtt 120

gctgggacac ctgcagctcc cccaaaggct gtgctgaaac tcgagcccca gtggatcaac 180

gtgctccagg aggactctgt gactctgaca tgccggggga ctcacagccc tgagagcgac 240

tccattcagt ggttccacaa tgggaatctc attcccaccc acacgcagcc cagctacagg 300

ttcaaggcca acaacaatga cagcggggag tacacgtgcc agactggcca gaccagcctc 360

agcgaccctg tgcatctgac tgtgctttct gagtggctgg tgctccagac ccctcacctg 420

gagttccagg agggagaaac catcgtgctg aggtgccaca gctggaagga caagcctctg 480

gtcaaggtca cattcttcca gaatggaaaa tccaagaaat tttcccgttc ggatcccaac 540

ttctccatcc cacaagcaaa ccacagtcac agtggtgatt accactgcac aggaaacata 600

ggctacacgc tgtactcatc caagcctgtg accatcactg tccaagctcc cagctcttca 660

ccgatgggga tcattgtggc tgtggtcact gggattgctg tagcggccat tgttgctgct 720

gtagtggcct tgatctactg caggaaaaag cggatttcag ccaatcccac taatcctgat 780

gaggctgaca aagttggggc tgagaacaca atcacctatt cacttctcat gcacccggat 840

gctctggaag agcctgatga ccagaaccgt atttag 876

<210> 21
<211> 930
<212> DNA
<213> Homo sapiens

<400> 21
atgggaatcc tgtcattctt acctgtcctt gccactgaga gtgactgggc tgactgcaag 60

tccccccagc cttggggtca tatgcttctg tggacagctg tgctattcct ggctcctgtt 120

gctgggacac ctgctccccc aaaggctgtg ctgaaactcg agccccagtg gatcaacgtg 180

ctccaggagg actctgtgac tctgacatgc cgggggactc acagccctga gagcgactcc 240

attcagtggt tccacaatgg gaatctcatt cccacccaca cgcagcccag ctacaggttc 300

aaggccaaca acaatgacag cggggagtac acgtgccaga ctggccagac cagcctcagc 360

gaccctgtgc atctgactgt gctttctgag tggctggtgc tccagacccc tcacctggag 420

ttccaggagg gagaaaccat cgtgctgagg tgccacagct ggaaggacaa gcctctggtc 480

aaggtcacat tcttccagaa tggaaaatcc aagaaatttt cccgttcgga tcccaacttc 540

tccatcccac aagcaaacca cagtcacagt ggtgattacc actgcacagg aaacataggc 600

tacacgctgt actcatccaa gcctgtgacc atcactgtcc aagctcccag ctcttcaccg 660

atggggatca ttgtggctgt ggtcactggg attgctgtag cggccattgt tgctgctgta 720

gtggccttga tctactgcag gaaaaagcgg atttcagctc tcccaggata ccctgagtgc 780

agggaaatgg gagagaccct ccctgagaaa ccagccaatc ccactaatcc tgatgaggct 840

gacaaagttg gggctgagaa cacaatcacc tattcacttc tcatgcaccc ggatgctctg 900

gaagagcctg atgaccagaa ccgtatttag 930

<210> 22
<211> 972
<212> DNA
<213> Homo sapiens

<400> 22
atgggaatcc tgtcattctt acctgtcctt gccactgaga gtgactgggc tgactgcaag 60

tccccccagc cttggggtca tatgcttctg tggacagctg tgctattcct ggctcctgtt 120

gctgggacac ctgcagctcc cccaaaggct gtgctgaaac tcgagcccca gtggatcaac 180
gtgctccaag aggactctgt gactctgaca tgccggggga ctcacagccc tgagagcgac 240
tccattccgt ggttccacaa tgggaatctc attcccaccc acacgcagcc cagctacagg 300
ttcaaggcca acaacaatga cagcggggag tacacgtgcc agactggcca gaccagcctc 360
agcgaccctg tgcatctgac tgtgctttct gagtggctgg tgctccagac ccctcacctg 420
gagttccagg agggagaaac catcgtgctg aggtgccaca gctggaagga caagcctctg 480
gtcaaggtca cattcttcca gaatggaaaa tccaagaaat tttcccgttc ggatcccaac 540
ttctccatcc cacaagcaaa ccacagtcac agtggtgatt accactgcac aggaaacata 600
ggctacacgc tgtactcatc caagcctgtg accatcactg tccaagctcc cagctcttca 660
ccgatgggga tcattgtggc tgtggtcact gggattgctg tagcggccat tgttgctgct 720
gtagtggcct tgatctactg caggaaaaag cggatttcag ccaattccac tgatcctgtg 780
aaggctgccc aatttgagcc acctggacgt caaatgattg ccatcagaaa gagacaacct 840
gaagaaacca acaatgacta tgaaacagct gacggcggct acatgactct gaaccccagg 900
gcacctactg acgatgataa aaacatctac ctgactcttc ctcccaacga ccatgtcaac 960
agtaataact aa 972

<210> 23
<211> 831
<212> DNA
<213> Homo sapiens

<400> 23
atgggaatcc tgtcattctt acctgtcctt gccactgaga gtgactgggc tgactgcaag 60
tccccccagc cttggggtca tatgcttctg tggacagctg tgctattcct ggctcctgtt 120
gctgggacac ctgcagctcc cccaaaggct gtgctgaaac tcgagcccca gtggatcaac 180
gtgctccaag aggactctgt gactctgaca tgccggggga ctcacagccc tgagagcgac 240
tccattccgt ggttccacaa tgggaatctc attcccaccc acacgcagcc cagctacagg 300
ttcaaggcca acaacaatga cagcggggag tacacgtgcc agactggcca gaccagcctc 360
agcgaccctg tgcatctgac tgtgctttct gagtggctgg tgctccagac ccctcacctg 420
gagttccagg agggagaaac catcgtgctg aggtgccaca gctggaagga caagcctctg 480
gtcaaggtca cattcttcca gaatggaaaa tccaagaaat tttcccgttc ggatcccaac 540
ttctccatcc cacaagcaaa ccacagtcac agtggtgatt accactgcac aggaaacata 600
ggctacacgc tgtactcatc caagcctgtg accatcactg tccaagctcc cagctcttca 660
ccgatgggga tcattgtggc tgtggtcact gggattgctg tagcggccat tgttgctgct 720
gtagtggcct tgatctactg caggaaaaag cggatttcag ccaattccac tgatcctgtg 780
aaggctgccc aatttgagat gctttcctgc acccacctgg acgtcaaatg a 831

<210> 24
<211> 804

<212>  DNA
<213>  Homo sapiens

<400>  24
atgggaatcc tgtcattctt acctgtcctt gccactgaga gtgactgggc tgactgcaag      60

tccccccagc cttggggtca tatgcttctg tggacagctg tgctattcct ggctcctgtt     120

gctgggacac ctgcagctcc cccaaaggct gtgctgaaac tcgagcccca gtggatcaac     180

gtgctccaag aggactctgt gactctgaca tgccggggga ctcacagccc tgagagcgac     240

tccattccgt ggttccacaa tgggaatctc attcccaccc acacgcagcc cagctacagg     300

ttcaaggcca acaacaatga cagcggggag tacacgtgcc agactggcca gaccagcctc     360

agcgaccctg tgcatctgac tgtgctttct gagtggctgg tgctccagac ccctcacctg     420

gagttccagg agggagaaac catcgtgctg aggtgccaca gctggaagga caagcctctg     480

gtcaaggtca cattcttcca gaatggaaaa tccaagaaat tttcccgttc ggatcccaac     540

ttctccatcc cacaagcaaa ccacagtcac agtggtgatt accactgcac aggaaacata     600

ggctacacgc tgtactcatc caagcctgtg accatcactg tccaagctcc cagctcttca     660

ccgatgggga tcattgtggc tgtggtcact gggattgctg tagcggccat tgttgctgct     720

gtagtggcct tgatctactg caggaaaaag cggatttcag ccacctggac gtcaaatgat     780

tgccatcaga aagagacaac ctga                                            804

<210>  25
<211>  873
<212>  DNA
<213>  Homo sapiens

<400>  25
atgggtggag gggctgggga aaggctgttt acttcctcct gtctagtcgg tttggtccct      60

ttagggctcc ggatatcttt ggtgacttgt ccactccagt gtggcatcat gtggcagctg     120

ctcctcccaa ctgctctgct acttctagtt tcagctggca tgcggactga agatctccca     180

aaggctgtgg tgttcctgga gcctcaatgg tacagggtgc tcgagaagga cagtgtgact     240

ctgaagtgcc agggagccta ctcccctgag gacaattcca cacagtggtt tcacaatgag     300

agcctcatct caagccaggc ctcgagctac ttcattgacg ctgccacagt cgacgacagt     360

ggagagtaca ggtgccagac aaacctctcc accctcagtg acccggtgca gctagaagtc     420

catatcggct ggctgttgct ccaggcccct cggtgggtgt tcaaggagga agaccctatt     480

cacctgaggt gtcacagctg gaagaacact gctctgcata aggtcacata tttacagaat     540

ggcaaaggca ggaagtattt tcatcataat tctgacttct acattccaaa agccacactc     600

aaagacagcg gctcctactt ctgcaggggg ctttttggga gtaaaaatgt gtcttcagag     660

actgtgaaca tcaccatcac tcaaggtttg gcagtgtcaa ccatctcatc attctttcca     720

cctgggtacc aagtctcttt ctgcttggtg atggtactcc tttttgcagt ggacacagga     780

ctatatttct ctgtgaagac aaacattcga agctcaacaa gagactggaa ggaccataaa     840

tttaaatgga gaaaggaccc tcaagacaaa tga                                  873

<210> 26
<211> 702
<212> DNA
<213> Homo sapiens

<400> 26
atgtggcagc tgctcctccc aactgctctg ctacttctag tttcagctgg catgcggact 60

gaagatctcc caaaggctgt ggtgttcctg gagcctcaat ggtacagcgt gcttgagaag 120

gacagtgtga ctctgaagtg ccagggagcc tactcccctg aggacaattc cacacagtgg 180

tttcacaatg agaacctcat ctcaagccag gcctcgagct acttcattga cgctgccaca 240

gtcaacgaca gtggagagta caggtgccag acaaacctct ccaccctcag tgacccggtg 300

cagctagaag tccatatcgg ctggctgttg ctccaggccc ctcggtgggt gttcaaggag 360

gaagacccta ttcacctgag gtgtcacagc tggaagaaca ctgctctgca taaggtcaca 420

tatttacaga atggcaaaga caggaagtat tttcatcata attctgactt ccacattcca 480

aaagccacac tcaaagatag cggctcctac ttctgcaggg ggcttgttgg gagtaaaaat 540

gtgtcttcag agactgtgaa catcaccatc actcaaggtt tggcagtgtc aaccatctca 600

tcattctctc cacctgggta ccaagtctct ttctgcttgg tgatggtact cctttttgca 660

gtggacacag gactatattt ctctgtgaag acaaacattt ga 702

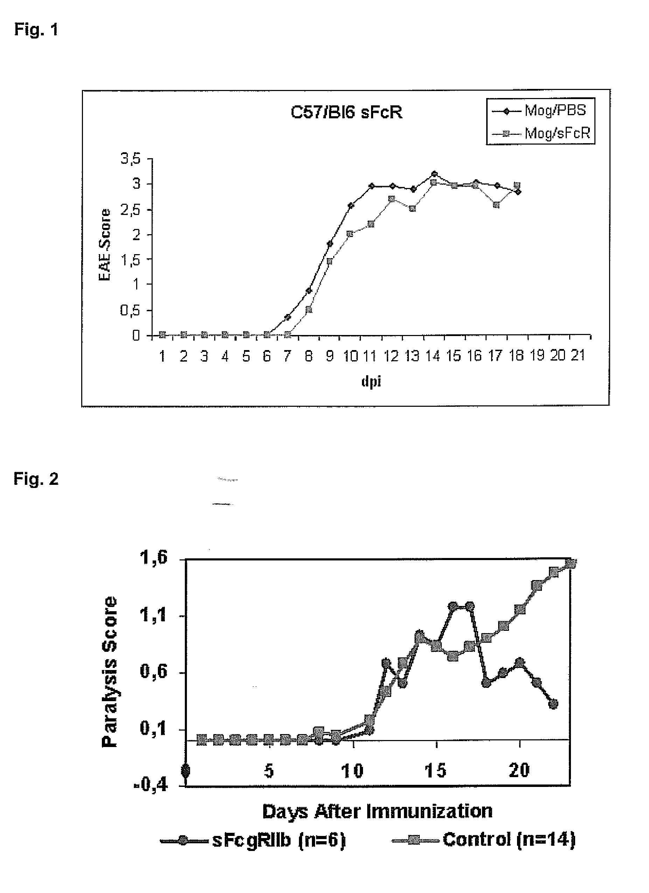

## Claims

1. Fcγ receptor (Fc-gamma receptor) for use in treating multiple sclerosis, wherein the multiple sclerosis is a B cell mediated form of multiple sclerosis and/or an autoantibody driven form of multiple sclerosis.

2. Fcγ receptor according to claim 1, wherein the B cell mediation of the multiple sclerosis and/or autoantibody driven form of multiple sclerosis is **characterized by** one or more of the following features,
a. the multiple sclerosis is ameliorated if the patient undergoes Intravenous immunoglobulin (IVIG) treatment and/or,
b. the multiple sclerosis is ameliorated if the patient undergoes anti-CD20 antibody treatment and/or,
c. the multiple sclerosis is ameliorated if the patient undergoes plasmapheresis and/or,
d. determining whether auto-antibodies against the antigen Myelin Oligodendrocyte Glycoprotein (MOG) are present in the patient and/or,
e. determining whether auto-antibodies against the antigen myelin basic protein (MBP) are present in the patient and/or,
f. determining whether auto-antibodies against aquaporin 4 are present in the patient.

3. Fcγ receptor according to claims 1 or 2, wherein the B cell mediation of the multiple sclerosis and/or autoantibody driven form of multiple sclerosis is determined prior to the use of the Fcγ receptor by means of one or more of the following tests,
a. determining whether the multiple sclerosis is ameliorated if the patient undergoes Intravenous immunoglobulin (IVIG) treatment and/or,
b. the multiple sclerosis is ameliorated if the patient undergoes anti-CD20 antibody treatment and/or,
c. the multiple sclerosis is ameliorated if the patient undergoes plasmapheresis and/or,
d. determining whether auto-antibodies against the antigen Myelin Oligodendrocyte Glycoprotein (MOG) are present in the patient and/or,
e. determining whether auto-antibodies against the antigen myelin basic protein (MBP) are present in the patient and/or,
f. determining whether auto-antibodies against aquaporin 4 are present in the patient.

4. Fcγ receptor according to claims 1 to 3, wherein the Fcγ is selected from the group of, FcγRI (CD64), FcγRIIA (CD32), FcγRIIB1 (CD32), FcγRIIB2 (CD32), FcγRIIc (CD32), FcγRIIIA (CD16) and FcγRIIIB (CD16).

5. Fcγ receptor according to claims 1 to 4, wherein the receptor lacks the transmebrane domain and/or the signal peptide and is soluble.

6. Fcγ receptor according to claims 1 to 5, wherein the receptor is chemically modified by PEGylation and/or affinity modulated.

7. Fcγ receptor according to claims 1 to 6, wherein the receptor is non glycosylated.

8. Fcγ receptor according to claims 1 to 7, wherein the receptor is FcγRIIB/C (CD32) or FcγRIIIA/B (CD16b).

9. Fcγ receptor according to claims 1 to 8, in an aqueous solution, wherein the amount to administered to a patient in a single dose is between 1 and 20 mg/kg.

10. Fcγ receptor according to claims 1 to 9, wherein the receptor has sequence selected from the group of, SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12 and SEQ ID NO. 13.

11. FcγR receptor according to claims 1 to 10, wherein the FcγR is a recombinant non-glycosylated human soluble FcγRIIb preferably selected from the group of SEQ ID NO. 5, SEQ ID NO. 6, SEQ ID NO. 7 and SEQ ID NO. 8.
